# EUROPEAN PATENT APPLICATION

(11) **EP 1 754 492 A1**
(43) Date of publication of application: **21.02.2007**
(21) Application number: 05745916.6
(22) Date of filing: 26.05.2005
(51) Int. Cl.: A61K 45/00, A61K 31/352, A61K 31/428, A61P 29/00, A61P 37/08, C07D 277/68, C07D 311/24, C07K 14/47, C12N 15/09

(54) **NOVEL DRUG DISCOVERY TARGET AND MEDICINE ACTING ON THE SAME**

(30) Priority: 26.05.2004 JP 2004156614
(71) Applicant: Reverse Proteomics Research Institute Co., Ltd, Chiyoda-ku Tokyo 101-0044 (JP)
(72) Inventor: TANAKA, Akito, c/o Astellas Pharma Inc., Tsukuba-shi, Ibaraki 305-8585 (JP); YAMAZAKI, Akira, c/o Dainippon Pharm. Co., Ltd., Suita-shi, Osaka 5640053 (JP); NAKANISHI, Akinobu, c/o Teijin Pharma Limited, Hino-shi, Tokyo 191-8512 (JP); TAKEUCHI, Mikio, c/o Astellas Pharma Inc., Tsukuba-shi, Ibaraki 305-8585 (JP); HARAMURA, Masayuki, Chugai Pharm. Co., Ltd., Kamakura-shi, Kanagawa 247-8530 (JP)
(74) Representative: Duckworth, Timothy John
(86) International application number: PCT/JP2005/010105
(87) International publication number: WO 2005/115468

(57) **Abstract**

The present invention provides a pharmaceutical composition comprising as an active ingredient a compound that specifically binds to MFP-2 or a functional fragment thereof and a screening method for the compound; the compound and a pharmaceutical composition comprising the same are highly useful as anti-inflammatory agents and anti-allergic agents.

SEQUENCE LISTING

<110> REVERSE PROTEOMICS RESEARCH INSTITUTE CO., LTD.

<120> Novel target for drug discovery and pharmaceutical agent which acts thereon

<130> 09737

<150> JP2004-156614
<151> 2004-05-26

<160> 6

<170> PatentIn version 3.1

<210> 1
<211> 2322
<212> DNA
<213> Rattus norvegicus

<220>
<221> CDS
<222> (28)..(2232)
<223>

<400> 1
gtgtgtgcgt ggtgcaggat agactca atg tcg cct ctg agg ttc gac ggg cgt 54
Met Ser Pro Leu Arg Phe Asp Gly Arg
1               5

gtg ggc ctg gtc acc ggc gcc ggg gga ggg ttg ggc aga gct tat ggg 102
Val Gly Leu Val Thr Gly Ala Gly Gly Gly Leu Gly Arg Ala Tyr Gly
10                  15                  20                  25

ctg gct ttt gca gaa aga gga gca tta gtt gtt gtg aat gac tta gga 150
Leu Ala Phe Ala Glu Arg Gly Ala Leu Val Val Val Asn Asp Leu Gly

                30                  35                  40

ggg gac ttc aaa ggc gtt ggg aaa ggc tct tct gcc gca gac aag gtc 198
Gly Asp Phe Lys Gly Val Gly Lys Gly Ser Ser Ala Ala Asp Lys Val
            45                  50                  55

gtg gaa gaa ata aga agg aga ggc ggg aaa gcg gtg gcc aat tac gat 246
Val Glu Glu Ile Arg Arg Arg Gly Gly Lys Ala Val Ala Asn Tyr Asp
        60                  65                  70

tca gtc gaa gca ggc gag aag ctt gtg aag aca gca ctg gac aca ttc 294
Ser Val Glu Ala Gly Glu Lys Leu Val Lys Thr Ala Leu Asp Thr Phe
    75                  80                  85

ggc aga ata gat gtt gtg gtg aac aat gct ggg atc ctg agg gac cct 342
Gly Arg Ile Asp Val Val Val Asn Asn Ala Gly Ile Leu Arg Asp Pro
90                  95                  100                 105

tcc ttc tct agg ata agt gat gaa gac tgg gat ata att caa aga gtt 390
Ser Phe Ser Arg Ile Ser Asp Glu Asp Trp Asp Ile Ile Gln Arg Val
                110                 115                 120

cat ttg cgg ggc tcc ttc caa gtg acc cgg gca gca tgg gat cat atg 438
His Leu Arg Gly Ser Phe Gln Val Thr Arg Ala Ala Trp Asp His Met
            125                 130                 135

aag aag cag aat tat gga aga atc att atg acg gcc tca gct tct gga 486
Lys Lys Gln Asn Tyr Gly Arg Ile Ile Met Thr Ala Ser Ala Ser Gly
        140                 145                 150

ata tac ggc aac ttt ggc cag gca aat tat agt gct gca aag ctg ggc 534
Ile Tyr Gly Asn Phe Gly Gln Ala Asn Tyr Ser Ala Ala Lys Leu Gly
    155                 160                 165

ctt ctg ggt ctc gcc aat act ctc gtg att gaa ggc agg aag aac aac 582
Leu Leu Gly Leu Ala Asn Thr Leu Val Ile Glu Gly Arg Lys Asn Asn

170                 175                 180                 185

att cat tgt aac acc att gcc cca aac gct ggg tca cgg atg aca gag     630
Ile His Cys Asn Thr Ile Ala Pro Asn Ala Gly Ser Arg Met Thr Glu
                190                 195                 200

acg gtg atg cca gaa gac ctc gtt gaa gcc ctg aag cca gag tat gtg     678
Thr Val Met Pro Glu Asp Leu Val Glu Ala Leu Lys Pro Glu Tyr Val
            205                 210                 215

gca ccg ctg gtc ctt tgg ctt tgc cat gag agc tgt gag gaa aat ggt     726
Ala Pro Leu Val Leu Trp Leu Cys His Glu Ser Cys Glu Glu Asn Gly
        220                 225                 230

ggc ttg ttt gag gtt gga gca gga tgg att gga aaa ttg cgc tgg gag     774
Gly Leu Phe Glu Val Gly Ala Gly Trp Ile Gly Lys Leu Arg Trp Glu
    235                 240                 245

agg acc ctg gga gcc att gtc agg aag cgg aat cag ccc atg act ccc     822
Arg Thr Leu Gly Ala Ile Val Arg Lys Arg Asn Gln Pro Met Thr Pro
250                 255                 260                 265

gag gca gtg agg gac aac tgg gtg aag atc tgt gac ttc agc aat gcc     870
Glu Ala Val Arg Asp Asn Trp Val Lys Ile Cys Asp Phe Ser Asn Ala
                270                 275                 280

agc gag ccg aag agc att caa gag tcc aca ggt ggt ata atc gaa gtt     918
Ser Glu Pro Lys Ser Ile Gln Glu Ser Thr Gly Gly Ile Ile Glu Val
            285                 290                 295

tta cat aaa ata gat tca gaa gga atc tca caa aat cac acc ggt caa     966
Leu His Lys Ile Asp Ser Glu Gly Ile Ser Gln Asn His Thr Gly Gln
        300                 305                 310

gtg gca tct gca gat gca tca gga ttt gct ggc gtc gtt ggc cac aaa     1014
Val Ala Ser Ala Asp Ala Ser Gly Phe Ala Gly Val Val Gly His Lys

315                 320                 325

ctt cct tca ttt tct tct tca tat acg gaa ctg cag tgc att atg tat      1062
Leu Pro Ser Phe Ser Ser Ser Tyr Thr Glu Leu Gln Cys Ile Met Tyr
330                 335                 340                 345

gcc ctc gga gta gga gct tca gtc aaa aat cca aag gac ttg aag ttt      1110
Ala Leu Gly Val Gly Ala Ser Val Lys Asn Pro Lys Asp Leu Lys Phe
                350                 355                 360

gtt tat gaa ggg agt cct gac ttc tcc tgt ttg cct aca att gga gtc      1158
Val Tyr Glu Gly Ser Pro Asp Phe Ser Cys Leu Pro Thr Ile Gly Val
            365                 370                 375

att gtc gct cag aag tcc ttg atg agt gga ggc tta gca gag gtt cct      1206
Ile Val Ala Gln Lys Ser Leu Met Ser Gly Gly Leu Ala Glu Val Pro
        380                 385                 390

ggg ctg tca atc aac ttt gca aag gtt ctt cat ggg gag cag tac ttg      1254
Gly Leu Ser Ile Asn Phe Ala Lys Val Leu His Gly Glu Gln Tyr Leu
    395                 400                 405

gag ttg tat aag cca ctt ccc cga tca ggg gaa tta aaa tgt gaa gca      1302
Glu Leu Tyr Lys Pro Leu Pro Arg Ser Gly Glu Leu Lys Cys Glu Ala
410                 415                 420                 425

gtt att gct gac atc ctg gat aaa ggc tct ggc ata gtg att gtt atg      1350
Val Ile Ala Asp Ile Leu Asp Lys Gly Ser Gly Ile Val Ile Val Met
                430                 435                 440

gac gtc tat tct tat tct ggc aag gaa ctt ata tgc tat aat cag ttc      1398
Asp Val Tyr Ser Tyr Ser Gly Lys Glu Leu Ile Cys Tyr Asn Gln Phe
            445                 450                 455

tct gtc ttc gtt gtt ggc tct gga ggc ttt ggt gga aaa cgg aca tca      1446
Ser Val Phe Val Val Gly Ser Gly Gly Phe Gly Gly Lys Arg Thr Ser

        460                 465                 470

gaa aaa ctc aaa gca gct gta gcc gta cca agt cgg cct cca gat gct 1494
Glu Lys Leu Lys Ala Ala Val Ala Val Pro Ser Arg Pro Pro Asp Ala
    475                 480                 485

gta ctg aga gat acc act tca gtg aat cag gcc gct ctg tac cgc ctc 1542
Val Leu Arg Asp Thr Thr Ser Val Asn Gln Ala Ala Leu Tyr Arg Leu
490                 495                 500                 505

agt gga gac tcg aat cct tta cac att gac ccg agc ttt gca ggc att 1590
Ser Gly Asp Ser Asn Pro Leu His Ile Asp Pro Ser Phe Ala Gly Ile
                510                 515                 520

gcc ggt ttt gag aaa ccc ata tta cac gga tta tgt act ttt ggg ttt 1638
Ala Gly Phe Glu Lys Pro Ile Leu His Gly Leu Cys Thr Phe Gly Phe
            525                 530                 535

tct gca agg cat gtt tta cag cag ttt gcg gat aat gat gtg tca aga 1686
Ser Ala Arg His Val Leu Gln Gln Phe Ala Asp Asn Asp Val Ser Arg
        540                 545                 550

ttc aag gcc att aag gtt cgt ttt gcc aaa cca gtg tat cca gga caa 1734
Phe Lys Ala Ile Lys Val Arg Phe Ala Lys Pro Val Tyr Pro Gly Gln
    555                 560                 565

act cta caa act gag atg tgg aag gaa gga aac aga att cat ttt caa 1782
Thr Leu Gln Thr Glu Met Trp Lys Glu Gly Asn Arg Ile His Phe Gln
570                 575                 580                 585

acc aag gtc caa gag act gga gac att gtc att tcc aat gca tat gtg 1830
Thr Lys Val Gln Glu Thr Gly Asp Ile Val Ile Ser Asn Ala Tyr Val
                590                 595                 600

gat ctt gtt cct aca tct gga gtt tcc gct cag aca cct tct gag ggt 1878
Asp Leu Val Pro Thr Ser Gly Val Ser Ala Gln Thr Pro Ser Glu Gly

605                 610                 615

gga gca ctg cag agt gct ctt gta ttt ggg gaa ata ggt cga cgc ctc      1926
Gly Ala Leu Gln Ser Ala Leu Val Phe Gly Glu Ile Gly Arg Arg Leu
        620                 625                 630

aag gat gtt gga cgt gag gtg gta aag aaa gta aat gct gta ttt gaa      1974
Lys Asp Val Gly Arg Glu Val Val Lys Lys Val Asn Ala Val Phe Glu
    635                 640                 645

tgg cat atc acg aaa aat ggg aat gtt gca gcc aag tgg acc att gac      2022
Trp His Ile Thr Lys Asn Gly Asn Val Ala Ala Lys Trp Thr Ile Asp
650                 655                 660                 665

ctg aag aac ggc tct gga gag gtt tac caa ggc cct gcc aaa ggc tct      2070
Leu Lys Asn Gly Ser Gly Glu Val Tyr Gln Gly Pro Ala Lys Gly Ser
                670                 675                 680

gct gac acg acc atc aca att tct gat gag gat ttc atg gaa gtg gtc      2118
Ala Asp Thr Thr Ile Thr Ile Ser Asp Glu Asp Phe Met Glu Val Val
            685                 690                 695

ctg ggc aag ctt aac cca cag aat gcc ttc ttc agt ggc aga ctg aag      2166
Leu Gly Lys Leu Asn Pro Gln Asn Ala Phe Phe Ser Gly Arg Leu Lys
        700                 705                 710

gcc cga gga aac atc atg ctg agc cag aag cta cag atg att ctg aaa      2214
Ala Arg Gly Asn Ile Met Leu Ser Gln Lys Leu Gln Met Ile Leu Lys
    715                 720                 725

gac tat gcc aag ctc tga aggacccact gcgtgcttta ataaaaccag             2262
Asp Tyr Ala Lys Leu
730

aatcattacg ttctgtctac gcagtcatgc tccagccttc tttgaaacga tccacggtaa    2322

<210> 2
<211> 734
<212> PRT
<213> Rattus norvegicus

<400> 2

Met Ser Pro Leu Arg Phe Asp Gly Arg Val Gly Leu Val Thr Gly Ala
1               5                   10                  15

Gly Gly Gly Leu Gly Arg Ala Tyr Gly Leu Ala Phe Ala Glu Arg Gly
            20                  25                  30

Ala Leu Val Val Val Asn Asp Leu Gly Gly Asp Phe Lys Gly Val Gly
        35                  40                  45

Lys Gly Ser Ser Ala Ala Asp Lys Val Val Glu Glu Ile Arg Arg Arg
    50                  55                  60

Gly Gly Lys Ala Val Ala Asn Tyr Asp Ser Val Glu Ala Gly Glu Lys
65                  70                  75                  80

Leu Val Lys Thr Ala Leu Asp Thr Phe Gly Arg Ile Asp Val Val Val
                85                  90                  95

Asn Asn Ala Gly Ile Leu Arg Asp Pro Ser Phe Ser Arg Ile Ser Asp
            100                 105                 110

Glu Asp Trp Asp Ile Ile Gln Arg Val His Leu Arg Gly Ser Phe Gln
        115                 120                 125

Val Thr Arg Ala Ala Trp Asp His Met Lys Lys Gln Asn Tyr Gly Arg
    130                 135                 140

Ile Ile Met Thr Ala Ser Ala Ser Gly Ile Tyr Gly Asn Phe Gly Gln

145                 150                 155                 160

Ala Asn Tyr Ser Ala Ala Lys Leu Gly Leu Leu Gly Leu Ala Asn Thr
                165                 170                 175

Leu Val Ile Glu Gly Arg Lys Asn Asn Ile His Cys Asn Thr Ile Ala
            180                 185                 190

Pro Asn Ala Gly Ser Arg Met Thr Glu Thr Val Met Pro Glu Asp Leu
        195                 200                 205

Val Glu Ala Leu Lys Pro Glu Tyr Val Ala Pro Leu Val Leu Trp Leu
    210                 215                 220

Cys His Glu Ser Cys Glu Glu Asn Gly Gly Leu Phe Glu Val Gly Ala
225                 230                 235                 240

Gly Trp Ile Gly Lys Leu Arg Trp Glu Arg Thr Leu Gly Ala Ile Val
                245                 250                 255

Arg Lys Arg Asn Gln Pro Met Thr Pro Glu Ala Val Arg Asp Asn Trp
            260                 265                 270

Val Lys Ile Cys Asp Phe Ser Asn Ala Ser Glu Pro Lys Ser Ile Gln
        275                 280                 285

Glu Ser Thr Gly Gly Ile Ile Glu Val Leu His Lys Ile Asp Ser Glu
    290                 295                 300

Gly Ile Ser Gln Asn His Thr Gly Gln Val Ala Ser Ala Asp Ala Ser
305                 310                 315                 320

Gly Phe Ala Gly Val Val Gly His Lys Leu Pro Ser Phe Ser Ser Ser
                325                 330                 335

Tyr Thr Glu Leu Gln Cys Ile Met Tyr Ala Leu Gly Val Gly Ala Ser

            340                 345                 350

Val Lys Asn Pro Lys Asp Leu Lys Phe Val Tyr Glu Gly Ser Pro Asp
        355                 360                 365

Phe Ser Cys Leu Pro Thr Ile Gly Val Ile Val Ala Gln Lys Ser Leu
    370                 375                 380

Met Ser Gly Gly Leu Ala Glu Val Pro Gly Leu Ser Ile Asn Phe Ala
385                 390                 395                 400

Lys Val Leu His Gly Glu Gln Tyr Leu Glu Leu Tyr Lys Pro Leu Pro
                405                 410                 415

Arg Ser Gly Glu Leu Lys Cys Glu Ala Val Ile Ala Asp Ile Leu Asp
            420                 425                 430

Lys Gly Ser Gly Ile Val Ile Val Met Asp Val Tyr Ser Tyr Ser Gly
        435                 440                 445

Lys Glu Leu Ile Cys Tyr Asn Gln Phe Ser Val Phe Val Val Gly Ser
    450                 455                 460

Gly Gly Phe Gly Gly Lys Arg Thr Ser Glu Lys Leu Lys Ala Ala Val
465                 470                 475                 480

Ala Val Pro Ser Arg Pro Pro Asp Ala Val Leu Arg Asp Thr Thr Ser
                485                 490                 495

Val Asn Gln Ala Ala Leu Tyr Arg Leu Ser Gly Asp Ser Asn Pro Leu
            500                 505                 510

His Ile Asp Pro Ser Phe Ala Gly Ile Ala Gly Phe Glu Lys Pro Ile
        515                 520                 525

Leu His Gly Leu Cys Thr Phe Gly Phe Ser Ala Arg His Val Leu Gln

530                 535                 540

Gln Phe Ala Asp Asn Asp Val Ser Arg Phe Lys Ala Ile Lys Val Arg
545                 550                 555                 560

Phe Ala Lys Pro Val Tyr Pro Gly Gln Thr Leu Gln Thr Glu Met Trp
                565                 570                 575

Lys Glu Gly Asn Arg Ile His Phe Gln Thr Lys Val Gln Glu Thr Gly
            580                 585                 590

Asp Ile Val Ile Ser Asn Ala Tyr Val Asp Leu Val Pro Thr Ser Gly
        595                 600                 605

Val Ser Ala Gln Thr Pro Ser Glu Gly Gly Ala Leu Gln Ser Ala Leu
    610                 615                 620

Val Phe Gly Glu Ile Gly Arg Arg Leu Lys Asp Val Gly Arg Glu Val
625                 630                 635                 640

Val Lys Lys Val Asn Ala Val Phe Glu Trp His Ile Thr Lys Asn Gly
                645                 650                 655

Asn Val Ala Ala Lys Trp Thr Ile Asp Leu Lys Asn Gly Ser Gly Glu
            660                 665                 670

Val Tyr Gln Gly Pro Ala Lys Gly Ser Ala Asp Thr Thr Ile Thr Ile
        675                 680                 685

Ser Asp Glu Asp Phe Met Glu Val Val Leu Gly Lys Leu Asn Pro Gln
    690                 695                 700

Asn Ala Phe Phe Ser Gly Arg Leu Lys Ala Arg Gly Asn Ile Met Leu
705                 710                 715                 720

Ser Gln Lys Leu Gln Met Ile Leu Lys Asp Tyr Ala Lys Leu

725                 730

<210>  3
<211>  2593
<212>  DNA
<213>  Homo sapiens

<220>
<221>  CDS
<222>  (49)..(2259)
<223>

<400>  3
ggccagcgcg tctgcttgtt cgtgtgtgtg tcgttgcagg ccttattc atg ggc tca      57
ccg ctg agg ttc gac ggg cgg gtg gta ctg gtc acc ggc gcg ggg gca      105
gga ttg ggc cga gcc tat gcc ctg gct ttt gca gaa aga gga gcg tta      153
gtt gtt gtg aat gat ttg gga ggg gac ttc aaa gga gtt ggt aaa ggc      201
tcc tta gct gct gat aag gtt gtt gaa gaa ata aga agg aga ggt gga      249
aaa gca gtg gcc aac tat gat tca gtg gaa gaa gga gag aag gtt gtg      297
aag aca gcc ctg gat gct ttt gga aga ata gat gtt gtg gtc aac aat 345
gct gga att ctg agg gat cgt tcc ttt gct agg ata agt gat gaa gac 393
tgg gat ata atc cac aga gtt cat ttg cgg ggt tca ttc caa gtg aca 441
cgg gca gca tgg gaa cac atg aag aaa cag aag tat gga agg att att 489
atg act tca tca gct tca gga ata tat ggc aac ttt ggc cag gcc aat 537
tat agt gct gca aag ttg ggt ctt ctg ggc ctt gca aat tct ctt gca 585
att gaa ggc agg aaa agc aac att cat tgt aac acc att gct cct aat 633
gcg gga tca cgg atg act cag aca gtt atg cct gaa gat ctt gtg gaa 681
gcc ctg aag cca gag tat gtg gca cct ctt gtc ctt tgg ctt tgt cac 729
gag agt tgt gag gag aat ggt ggc ttg ttt gag gtt gga gca gga tgg 777
att gga aaa tta cgc tgg gag cgg act ctt gga gct att gta aga caa 825
aag aat cac cca atg act cct gag gca gtc aag gct aac tgg aag aag 873
atc tgt gac ttt gag aat gcc agc aag cct cag agt atc caa gaa tca 921
act ggc agt ata att gaa gtt ctg agt aaa ata gat tca gaa gga gga 969
gtt tca gca aat cat act agt cgt gca acg tct aca gca aca tca gga 1017
ttt gct gga gct att ggc cag aaa ctc cct cca ttt tct tat gct tat 1065
acg gaa ctg gaa gct att atg tat gcc ctt gga gtg gga gcg tca atc 1113
aag gat cca aaa gat ttg aaa ttt att tat gaa gga agt tct gat ttc 1161
tcc tgt ttg ccc acc ttc gga gtt atc ata ggt cag aaa tct atg atg 1209
ggt gga gga tta gca gaa att cct gga ctt tca atc aac ttt gca aag 1257
gtt ctt cat gga gag cag tac tta gag tta tat aaa cca ctt ccc aga 1305
gca gga aaa tta aaa tgt gaa gca gtt gtt gct gat gtc cta gat aaa 1353
gga tcc ggt gta gtg att att atg gat gtc tat tct tat tct gag aag 1401
gaa ctt ata tgc cac aat cag ttc tct ctc ttt ctt gtt ggc tct gga 1449
ggc ttt ggt gga aaa cgg aca tca gac aaa gtc aag gta gct gta gcc 1497
ata cct aat aga cct cct gat gct gta ctt aca gat acc acc tct ctt 1545
aat cag gct gct ttg tac cgc ctc agt gga gac tgg aat ccc tta cac 1593
att gat cct aac ttt gct agt cta gca ggt ttt gac aag ccc ata tta 1641
cat gga tta tgt aca ttt gga ttt tct gcc agg cgt gtg tta cag cag 1689
ttt gca gat aat gat gtg tca aga ttc aag gca att aag gct cgt ttt 1737
gca aaa cca gta tat cca gga caa act cta caa act gag atg tgg aag 1785
gaa gga aac aga att cat ttt caa acc aag gtc caa gaa act gga gac 1833
att gtc att tca aat gca tat gtg gat ctt gca cca aca tct ggt act 1881
tca gct aag aca ccc tct gag ggc ggg aag ctt cag agt acc ttt gta 1929
ttt gag gaa ata gga cgc cgc cta aag gat att ggg cct gag gtg gtg 1977
aag aaa gta aat gct gta ttt gag tgg cat ata acc aaa ggc gga aat 2025
att ggg gct aag tgg act att gac ctg aaa agt ggt tct gga aaa gtg     2073
tac caa ggc cct gca aaa ggt gct gct gat aca aca atc ata ctt tca     2121
gat gaa gat ttc atg gag gtg gtc ctg ggc aag ctt gac cct cag aag     2169
gca ttc ttt agt ggc agg ctg aag gcc aga ggg aac atc atg ctg agc     2217
cag aaa ctt cag atg att ctt aaa gac tac gcc aag ctc tga             2259
agggcacact acactattaa taaaaatgga atcattaaat actctcttca cccaaatatg   2319
cttgattatt ctgcaaaagt gattagaact aagatgcagg ggaaattgct taacattttc   2379
agatatcaga taactgcaga ttttcatttt ctactaattt tcatgtatca ttatttttac   2439
aaggaactat atataagcta gcacatgatt atccttctgt tcttagatct gtatcttcat   2499
aataaaaaat tttgcccaag tcctgtttcc ttagaatttg tgatagcatt gataagttga   2559
aaggaaaatt aaatcaataa aggcctttga tacc                               2593

<210>  4
<211>  736
<212>  PRT
<213>  Homo sapiens

<400>  4

<210>  5
<211>  2468
<212>  DNA
<213>  Mus musculus

<220>
<221>  CDS

<222>  (13)..(2220)
<223>

<400>  5
caggctgagc tc atg gct tcc ccc ctg agg ttc gac ggg cgt gtg gtc ttg      51
              Met Ala Ser Pro Leu Arg Phe Asp Gly Arg Val Val Leu
              1               5                   10

gtc acc ggc ccc ggg gga gga ttg ggc cga gct tac gcc ctg gcg ttt      99
Val Thr Gly Pro Gly Gly Gly Leu Gly Arg Ala Tyr Ala Leu Ala Phe
    15                  20                  25

gca gaa aga gga gca tta gtc att gtg aac gac tta gga ggg gac ttc      147
Ala Glu Arg Gly Ala Leu Val Ile Val Asn Asp Leu Gly Gly Asp Phe
30                  35                  40                  45

aag gga att ggt aaa ggc tcc tct gct gca gac aag gtt gtg gca gag      195
Lys Gly Ile Gly Lys Gly Ser Ser Ala Ala Asp Lys Val Val Ala Glu
                50                  55                  60

ata aga agg aaa ggc gga aaa gca gtg gcc aat tac gat tca gtt gaa      243
Ile Arg Arg Lys Gly Gly Lys Ala Val Ala Asn Tyr Asp Ser Val Glu
            65                  70                  75

gca ggc gag aag ctt gtg aag acg gca ctg gac aca ttt ggc aga ata      291
Ala Gly Glu Lys Leu Val Lys Thr Ala Leu Asp Thr Phe Gly Arg Ile
        80                  85                  90

gac gtt gtg gtc aac aat gct gga atc ctg agg gac cgt tcc ttc tcc      339
Asp Val Val Val Asn Asn Ala Gly Ile Leu Arg Asp Arg Ser Phe Ser
    95                  100                 105

agg ata agt gat gaa gac tgg gat ata att cat aga gtt cat ttg cgg      387
Arg Ile Ser Asp Glu Asp Trp Asp Ile Ile His Arg Val His Leu Arg
110                 115                 120                 125

ggc tcc ttc caa gtg acc cgg gca gca tgg gac cat atg aag aaa cag 435
Gly Ser Phe Gln Val Thr Arg Ala Ala Trp Asp His Met Lys Lys Gln
                130                 135                 140

aat tat gga aga atc ctt atg act tcc tca gct tct gga ata tat ggc 483
Asn Tyr Gly Arg Ile Leu Met Thr Ser Ser Ala Ser Gly Ile Tyr Gly
            145                 150                 155

aac ttt ggc cag gcg aat tat agt gct gca aag ctg ggc att ctg ggt 531
Asn Phe Gly Gln Ala Asn Tyr Ser Ala Ala Lys Leu Gly Ile Leu Gly
        160                 165                 170

ctc tgc aat act ctc gcc att gaa ggc agg aag aac aac att cat tgc 579
Leu Cys Asn Thr Leu Ala Ile Glu Gly Arg Lys Asn Asn Ile His Cys
    175                 180                 185

aac acc att gcc ccc aac gct ggg tca cgg atg acg gag act gtg ttg 627
Asn Thr Ile Ala Pro Asn Ala Gly Ser Arg Met Thr Glu Thr Val Leu
190                 195                 200                 205

ccg gaa gat ctt gtt gaa gcc ctg aag cca gag tat gtg gcc cct ctg 675
Pro Glu Asp Leu Val Glu Ala Leu Lys Pro Glu Tyr Val Ala Pro Leu
                210                 215                 220

gtg ctt tgg ctt tgc cat gag agc tgt gag gaa aat ggt ggc cta ttt 723
Val Leu Trp Leu Cys His Glu Ser Cys Glu Glu Asn Gly Gly Leu Phe
            225                 230                 235

gag gtt gga gca gga tgg att gga aaa ttg cgc tgg gag agg acc ctg 771
Glu Val Gly Ala Gly Trp Ile Gly Lys Leu Arg Trp Glu Arg Thr Leu
        240                 245                 250

ggc gcc atc gtc aga aag cgg aat cag ccc atg act ccc gag gca gtg 819
Gly Ala Ile Val Arg Lys Arg Asn Gln Pro Met Thr Pro Glu Ala Val
    255                 260                 265

agg gac aac tgg gag aag atc tgt gac ttc agc aat gcc agc aag ccg 867
Arg Asp Asn Trp Glu Lys Ile Cys Asp Phe Ser Asn Ala Ser Lys Pro
270                 275                 280                 285

cag acc att caa gaa tca aca ggt ggt ata gtc gaa gtt tta cat aag 915
Gln Thr Ile Gln Glu Ser Thr Gly Gly Ile Val Glu Val Leu His Lys
                290                 295                 300

gta gat tca gaa gga atc tca cca aac cgt acc agt cac gcg gca cct 963
Val Asp Ser Glu Gly Ile Ser Pro Asn Arg Thr Ser His Ala Ala Pro
            305                 310                 315

gca gcc acg tca gga ttc gtt ggt gct gtt ggc cat aaa ctt cct tca 1011
Ala Ala Thr Ser Gly Phe Val Gly Ala Val Gly His Lys Leu Pro Ser
        320                 325                 330

ttt tct tct tcg tat acg gag ctg cag agt att atg tat gcc ctc gga 1059
Phe Ser Ser Ser Tyr Thr Glu Leu Gln Ser Ile Met Tyr Ala Leu Gly
    335                 340                 345

gtg gga gcg tca gtc aaa aat cca aag gat ttg aag ttt gtt tat gaa 1107
Val Gly Ala Ser Val Lys Asn Pro Lys Asp Leu Lys Phe Val Tyr Glu
350                 355                 360                 365

ggc agt gct gac ttc tcc tgt ttg ccc acc ttc gga gtc att gtc gct 1155
Gly Ser Ala Asp Phe Ser Cys Leu Pro Thr Phe Gly Val Ile Val Ala
                370                 375                 380

cag aag tcc atg atg aat gga ggg ctg gca gag gtt cct ggg ctg tca 1203
Gln Lys Ser Met Met Asn Gly Gly Leu Ala Glu Val Pro Gly Leu Ser
            385                 390                 395

ttc aac ttt gca aag gct ctt cac ggg gag cag tac ttg gag ctg tat 1251
Phe Asn Phe Ala Lys Ala Leu His Gly Glu Gln Tyr Leu Glu Leu Tyr
        400                 405                 410

aag cca ctt ctt cga tca gga gaa tta aaa tgt gaa gca gtt att gct 1299
Lys Pro Leu Leu Arg Ser Gly Glu Leu Lys Cys Glu Ala Val Ile Ala
    415                 420                 425

gac atc ctg gat aaa ggc tct ggc gta gtg att gtt atg gac gtc tat 1347
Asp Ile Leu Asp Lys Gly Ser Gly Val Val Ile Val Met Asp Val Tyr
430                 435                 440                 445

tct tat tct ggg aag gaa ctt ata tgc tat aat cag ttc tct gtc ttt 1395
Ser Tyr Ser Gly Lys Glu Leu Ile Cys Tyr Asn Gln Phe Ser Val Phe
                450                 455                 460

gtt gtt ggc tct ggg ggc ttt ggt gga aaa cgg aca tca gaa aaa ctc 1443
Val Val Gly Ser Gly Gly Phe Gly Gly Lys Arg Thr Ser Glu Lys Leu
            465                 470                 475

aaa gca gct gta gct gta cca aat cga cct cca gat gct gta ctg aga 1491
Lys Ala Ala Val Ala Val Pro Asn Arg Pro Pro Asp Ala Val Leu Arg
        480                 485                 490

gat gcc acc tca ctg aat cag gcc gcg ctg tac cgc ctc agc gga gac 1539
Asp Ala Thr Ser Leu Asn Gln Ala Ala Leu Tyr Arg Leu Ser Gly Asp
    495                 500                 505

tgg aat cct cta cac att gac ccg gac ttt gcg agc gtt gcc ggt ttt 1587
Trp Asn Pro Leu His Ile Asp Pro Asp Phe Ala Ser Val Ala Gly Phe
510                 515                 520                 525

gag aag ccc ata tta cat gga cta tgt acc ttt gga ttt tct gca agg 1635
Glu Lys Pro Ile Leu His Gly Leu Cys Thr Phe Gly Phe Ser Ala Arg
                530                 535                 540

cat gtt tta cag cag ttt gca gat aat gat gta tca aga ttc aag gcg 1683
His Val Leu Gln Gln Phe Ala Asp Asn Asp Val Ser Arg Phe Lys Ala
            545                 550                 555

att aag gtt cgt ttt gcc aaa cca gtg tat cca gga cag act cta caa 1731
Ile Lys Val Arg Phe Ala Lys Pro Val Tyr Pro Gly Gln Thr Leu Gln
        560                 565                 570

act gag atg tgg aag gaa gga aac aga att cat ttt caa acc aag gtc 1779
Thr Glu Met Trp Lys Glu Gly Asn Arg Ile His Phe Gln Thr Lys Val
    575                 580                 585

cac gag act gga gat gtt gtc att tca aat gcg tac gtg gat ctc gtg 1827
His Glu Thr Gly Asp Val Val Ile Ser Asn Ala Tyr Val Asp Leu Val
590                 595                 600                 605

cct gca tct gga gtt tca acc cag aca cct tca gag ggt gga gag ctc 1875
Pro Ala Ser Gly Val Ser Thr Gln Thr Pro Ser Glu Gly Gly Glu Leu
                610                 615                 620

cag agt gct ctt gtg ttt ggg gag ata ggc cgc cgc ctc aag agt gtt 1923
Gln Ser Ala Leu Val Phe Gly Glu Ile Gly Arg Arg Leu Lys Ser Val
            625                 630                 635

ggc cgt gag gtg gta aag aaa gcg aat gct gtg ttt gaa tgg cat atc 1971
Gly Arg Glu Val Val Lys Lys Ala Asn Ala Val Phe Glu Trp His Ile
        640                 645                 650

acg aaa ggt ggg act gtt gca gcc aag tgg acc att gac ctg aag agc 2019
Thr Lys Gly Gly Thr Val Ala Ala Lys Trp Thr Ile Asp Leu Lys Ser
    655                 660                 665

ggc tca ggg gag gtg tac caa ggc ccc gca aag ggc tct gct gat gtg 2067
Gly Ser Gly Glu Val Tyr Gln Gly Pro Ala Lys Gly Ser Ala Asp Val
670                 675                 680                 685

acc atc atc att tcc gat gag gat ttt atg gaa gtg gtc ttc ggc aag 2115
Thr Ile Ile Ile Ser Asp Glu Asp Phe Met Glu Val Val Phe Gly Lys
                690                 695                 700

ctt gac cca cag aag gcc ttc ttc agt ggc agg ctg aag gcc aga ggg     2163
Leu Asp Pro Gln Lys Ala Phe Phe Ser Gly Arg Leu Lys Ala Arg Gly
            705                 710                 715

aac atc atg ctg agc cag aaa cta cag atg att ctt aaa gac tat gcc     2211
Asn Ile Met Leu Ser Gln Lys Leu Gln Met Ile Leu Lys Asp Tyr Ala
        720                 725                 730

aag ctc tga agggaaccca ctgtgtgctg ttaaaggagt caataattaa             2260
Lys Leu
    735

atactgtcta cccagctgag ccgcagcctt ctgcgatcca caggagtgtg caggagaaat   2320
cgcttcacat ttccagattc agataacttg catattttca ttttctacta atttttcaca   2380
tatttttaca aggaactgta atctaggtag caaaataatc attctgttca tagatctgta   2440
tcttaataaa aaaaatcaac caaaaacc                                      2468

<210>  6
<211>  735
<212>  PRT
<213>  Mus musculus

<400>  6
Met Ala Ser Pro Leu Arg Phe Asp Gly Arg Val Val Leu Val Thr Gly
1               5                   10                  15

Pro Gly Gly Gly Leu Gly Arg Ala Tyr Ala Leu Ala Phe Ala Glu Arg
            20                  25                  30

Gly Ala Leu Val Ile Val Asn Asp Leu Gly Gly Asp Phe Lys Gly Ile
        35                  40                  45

Gly Lys Gly Ser Ser Ala Ala Asp Lys Val Val Ala Glu Ile Arg Arg
    50                  55                  60

Lys Gly Gly Lys Ala Val Ala Asn Tyr Asp Ser Val Glu Ala Gly Glu

65                    70                    75                    80

Lys Leu Val Lys Thr Ala Leu Asp Thr Phe Gly Arg Ile Asp Val Val
                85                    90                    95

Val Asn Asn Ala Gly Ile Leu Arg Asp Arg Ser Phe Ser Arg Ile Ser
            100                   105                   110

Asp Glu Asp Trp Asp Ile Ile His Arg Val His Leu Arg Gly Ser Phe
        115                   120                   125

Gln Val Thr Arg Ala Ala Trp Asp His Met Lys Lys Gln Asn Tyr Gly
    130                   135                   140

Arg Ile Leu Met Thr Ser Ser Ala Ser Gly Ile Tyr Gly Asn Phe Gly
145                   150                   155                   160

Gln Ala Asn Tyr Ser Ala Ala Lys Leu Gly Ile Leu Gly Leu Cys Asn
                165                   170                   175

Thr Leu Ala Ile Glu Gly Arg Lys Asn Asn Ile His Cys Asn Thr Ile
            180                   185                   190

Ala Pro Asn Ala Gly Ser Arg Met Thr Glu Thr Val Leu Pro Glu Asp
        195                   200                   205

Leu Val Glu Ala Leu Lys Pro Glu Tyr Val Ala Pro Leu Val Leu Trp
    210                   215                   220

Leu Cys His Glu Ser Cys Glu Glu Asn Gly Gly Leu Phe Glu Val Gly
225                   230                   235                   240

Ala Gly Trp Ile Gly Lys Leu Arg Trp Glu Arg Thr Leu Gly Ala Ile
                245                   250                   255

Val Arg Lys Arg Asn Gln Pro Met Thr Pro Glu Ala Val Arg Asp Asn

            260                 265                 270

Trp Glu Lys Ile Cys Asp Phe Ser Asn Ala Ser Lys Pro Gln Thr Ile
        275                 280                 285

Gln Glu Ser Thr Gly Gly Ile Val Glu Val Leu His Lys Val Asp Ser
    290                 295                 300

Glu Gly Ile Ser Pro Asn Arg Thr Ser His Ala Ala Pro Ala Ala Thr
305                 310                 315                 320

Ser Gly Phe Val Gly Ala Val Gly His Lys Leu Pro Ser Phe Ser Ser
                325                 330                 335

Ser Tyr Thr Glu Leu Gln Ser Ile Met Tyr Ala Leu Gly Val Gly Ala
            340                 345                 350

Ser Val Lys Asn Pro Lys Asp Leu Lys Phe Val Tyr Glu Gly Ser Ala
        355                 360                 365

Asp Phe Ser Cys Leu Pro Thr Phe Gly Val Ile Val Ala Gln Lys Ser
    370                 375                 380

Met Met Asn Gly Gly Leu Ala Glu Val Pro Gly Leu Ser Phe Asn Phe
385                 390                 395                 400

Ala Lys Ala Leu His Gly Glu Gln Tyr Leu Glu Leu Tyr Lys Pro Leu
                405                 410                 415

Leu Arg Ser Gly Glu Leu Lys Cys Glu Ala Val Ile Ala Asp Ile Leu
            420                 425                 430

Asp Lys Gly Ser Gly Val Val Ile Val Met Asp Val Tyr Ser Tyr Ser
        435                 440                 445

Gly Lys Glu Leu Ile Cys Tyr Asn Gln Phe Ser Val Phe Val Val Gly

    450                 455                 460

Ser Gly Gly Phe Gly Gly Lys Arg Thr Ser Glu Lys Leu Lys Ala Ala
465                 470                 475                 480

Val Ala Val Pro Asn Arg Pro Pro Asp Ala Val Leu Arg Asp Ala Thr
                485                 490                 495

Ser Leu Asn Gln Ala Ala Leu Tyr Arg Leu Ser Gly Asp Trp Asn Pro
            500                 505                 510

Leu His Ile Asp Pro Asp Phe Ala Ser Val Ala Gly Phe Glu Lys Pro
        515                 520                 525

Ile Leu His Gly Leu Cys Thr Phe Gly Phe Ser Ala Arg His Val Leu
    530                 535                 540

Gln Gln Phe Ala Asp Asn Asp Val Ser Arg Phe Lys Ala Ile Lys Val
545                 550                 555                 560

Arg Phe Ala Lys Pro Val Tyr Pro Gly Gln Thr Leu Gln Thr Glu Met
                565                 570                 575

Trp Lys Glu Gly Asn Arg Ile His Phe Gln Thr Lys Val His Glu Thr
            580                 585                 590

Gly Asp Val Val Ile Ser Asn Ala Tyr Val Asp Leu Val Pro Ala Ser
        595                 600                 605

Gly Val Ser Thr Gln Thr Pro Ser Glu Gly Gly Glu Leu Gln Ser Ala
    610                 615                 620

Leu Val Phe Gly Glu Ile Gly Arg Arg Leu Lys Ser Val Gly Arg Glu
625                 630                 635                 640

Val Val Lys Lys Ala Asn Ala Val Phe Glu Trp His Ile Thr Lys Gly

                645                 650                 655

Gly Thr Val Ala Ala Lys Trp Thr Ile Asp Leu Lys Ser Gly Ser Gly
            660                 665                 670

Glu Val Tyr Gln Gly Pro Ala Lys Gly Ser Ala Asp Val Thr Ile Ile
        675                 680                 685

Ile Ser Asp Glu Asp Phe Met Glu Val Val Phe Gly Lys Leu Asp Pro
    690                 695                 700

Gln Lys Ala Phe Phe Ser Gly Arg Leu Lys Ala Arg Gly Asn Ile Met
705                 710                 715                 720

Leu Ser Gln Lys Leu Gln Met Ile Leu Lys Asp Tyr Ala Lys Leu
                725                 730                 735

SEQUENCE LISTING

<110>  REVERSE PROTEOMICS RESEARCH INSTITUTE CO., LTD.

<120>  Novel drug discovery target and medicine acting on the same

<130>  N100133 TJD

<140>  EP05745916.6
<141>  2005-05-26

<150>  JP2004-156614
<151>  2004-05-26

<160>  6

<170>  PatentIn version 3.1

<210>  1
<211>  2322
<212>  DNA
<213>  Rattus norvegicus

<220>
<221>  CDS
<222>  (28)..(2232)
<223>

<400>  1

gtgtgtgcgt ggtgcaggat agactca atg tcg cct ctg agg ttc gac ggg cgt      54
                              Met Ser Pro Leu Arg Phe Asp Gly Arg
                              1               5

gtg ggc ctg gtc acc ggc gcc ggg gga ggg ttg ggc aga gct tat ggg      102
Val Gly Leu Val Thr Gly Ala Gly Gly Gly Leu Gly Arg Ala Tyr Gly
10                  15                  20                  25

ctg gct ttt gca gaa aga gga gca tta gtt gtt gtg aat gac tta gga      150
Leu Ala Phe Ala Glu Arg Gly Ala Leu Val Val Val Asn Asp Leu Gly
                30                  35                  40

ggg gac ttc aaa ggc gtt ggg aaa ggc tct tct gcc gca gac aag gtc      198
Gly Asp Phe Lys Gly Val Gly Lys Gly Ser Ser Ala Ala Asp Lys Val
            45                  50                  55

gtg gaa gaa ata aga agg aga ggc ggg aaa gcg gtg gcc aat tac gat      246
Val Glu Glu Ile Arg Arg Arg Gly Gly Lys Ala Val Ala Asn Tyr Asp
        60                  65                  70

tca gtc gaa gca ggc gag aag ctt gtg aag aca gca ctg gac aca ttc      294
Ser Val Glu Ala Gly Glu Lys Leu Val Lys Thr Ala Leu Asp Thr Phe

Ser Val Glu Ala Gly Glu Lys Leu Val Lys Thr Ala Leu Asp Thr Phe
    75                  80                  85

ggc aga ata gat gtt gtg gtg aac aat gct ggg atc ctg agg gac cct      342
Gly Arg Ile Asp Val Val Val Asn Asn Ala Gly Ile Leu Arg Asp Pro
90                  95                  100                 105

tcc ttc tct agg ata agt gat gaa gac tgg gat ata att caa aga gtt      390
Ser Phe Ser Arg Ile Ser Asp Glu Asp Trp Asp Ile Ile Gln Arg Val
                110                 115                 120

cat ttg cgg ggc tcc ttc caa gtg acc cgg gca gca tgg gat cat atg      438
His Leu Arg Gly Ser Phe Gln Val Thr Arg Ala Ala Trp Asp His Met
            125                 130                 135

aag aag cag aat tat gga aga atc att atg acg gcc tca gct tct gga      486
Lys Lys Gln Asn Tyr Gly Arg Ile Ile Met Thr Ala Ser Ala Ser Gly
        140                 145                 150

ata tac ggc aac ttt ggc cag gca aat tat agt gct gca aag ctg ggc      534
Ile Tyr Gly Asn Phe Gly Gln Ala Asn Tyr Ser Ala Ala Lys Leu Gly
    155                 160                 165

ctt ctg ggt ctc gcc aat act ctc gtg att gaa ggc agg aag aac aac      582
Leu Leu Gly Leu Ala Asn Thr Leu Val Ile Glu Gly Arg Lys Asn Asn
170                 175                 180                 185

att cat tgt aac acc att gcc cca aac gct ggg tca cgg atg aca gag      630
Ile His Cys Asn Thr Ile Ala Pro Asn Ala Gly Ser Arg Met Thr Glu
                190                 195                 200

acg gtg atg cca gaa gac ctc gtt gaa gcc ctg aag cca gag tat gtg      678
Thr Val Met Pro Glu Asp Leu Val Glu Ala Leu Lys Pro Glu Tyr Val
            205                 210                 215

gca ccg ctg gtc ctt tgg ctt tgc cat gag agc tgt gag gaa aat ggt      726
Ala Pro Leu Val Leu Trp Leu Cys His Glu Ser Cys Glu Glu Asn Gly
        220                 225                 230

ggc ttg ttt gag gtt gga gca gga tgg att gga aaa ttg cgc tgg gag      774
Gly Leu Phe Glu Val Gly Ala Gly Trp Ile Gly Lys Leu Arg Trp Glu
    235                 240                 245

agg acc ctg gga gcc att gtc agg aag cgg aat cag ccc atg act ccc      822
Arg Thr Leu Gly Ala Ile Val Arg Lys Arg Asn Gln Pro Met Thr Pro
250                 255                 260                 265

gag gca gtg agg gac aac tgg gtg aag atc tgt gac ttc agc aat gcc      870
Glu Ala Val Arg Asp Asn Trp Val Lys Ile Cys Asp Phe Ser Asn Ala
                270                 275                 280

agc gag ccg aag agc att caa gag tcc aca ggt ggt ata atc gaa gtt      918
Ser Glu Pro Lys Ser Ile Gln Glu Ser Thr Gly Gly Ile Ile Glu Val
            285                 290                 295

tta cat aaa ata gat tca gaa gga atc tca caa aat cac acc ggt caa 966
Leu His Lys Ile Asp Ser Glu Gly Ile Ser Gln Asn His Thr Gly Gln
300 305 310

gtg gca tct gca gat gca tca gga ttt gct ggc gtc gtt ggc cac aaa 1014
Val Ala Ser Ala Asp Ala Ser Gly Phe Ala Gly Val Val Gly His Lys
315 320 325

ctt cct tca ttt tct tct tca tat acg gaa ctg cag tgc att atg tat 1062
Leu Pro Ser Phe Ser Ser Ser Tyr Thr Glu Leu Gln Cys Ile Met Tyr
330 335 340 345

gcc ctc gga gta gga gct tca gtc aaa aat cca aag gac ttg aag ttt 1110
Ala Leu Gly Val Gly Ala Ser Val Lys Asn Pro Lys Asp Leu Lys Phe
350 355 360

gtt tat gaa ggg agt cct gac ttc tcc tgt ttg cct aca att gga gtc 1158
Val Tyr Glu Gly Ser Pro Asp Phe Ser Cys Leu Pro Thr Ile Gly Val
365 370 375

att gtc gct cag aag tcc ttg atg agt gga ggc tta gca gag gtt cct 1206
Ile Val Ala Gln Lys Ser Leu Met Ser Gly Gly Leu Ala Glu Val Pro
380 385 390

ggg ctg tca atc aac ttt gca aag gtt ctt cat ggg gag cag tac ttg 1254
Gly Leu Ser Ile Asn Phe Ala Lys Val Leu His Gly Glu Gln Tyr Leu
395 400 405

gag ttg tat aag cca ctt ccc cga tca ggg gaa tta aaa tgt gaa gca 1302
Glu Leu Tyr Lys Pro Leu Pro Arg Ser Gly Glu Leu Lys Cys Glu Ala
410 415 420 425

gtt att gct gac atc ctg gat aaa ggc tct ggc ata gtg att gtt atg 1350
Val Ile Ala Asp Ile Leu Asp Lys Gly Ser Gly Ile Val Ile Val Met
430 435 440

gac gtc tat tct tat tct ggc aag gaa ctt ata tgc tat aat cag ttc 1398
Asp Val Tyr Ser Tyr Ser Gly Lys Glu Leu Ile Cys Tyr Asn Gln Phe
445 450 455

tct gtc ttc gtt gtt ggc tct gga ggc ttt ggt gga aaa cgg aca tca 1446
Ser Val Phe Val Val Gly Ser Gly Gly Phe Gly Gly Lys Arg Thr Ser
460 465 470

gaa aaa ctc aaa gca gct gta gcc gta cca agt cgg cct cca gat gct 1494
Glu Lys Leu Lys Ala Ala Val Ala Val Pro Ser Arg Pro Pro Asp Ala
475 480 485

gta ctg aga gat acc act tca gtg aat cag gcc gct ctg tac cgc ctc 1542
Val Leu Arg Asp Thr Thr Ser Val Asn Gln Ala Ala Leu Tyr Arg Leu
490 495 500 505

agt gga gac tcg aat cct tta cac att gac ccg agc ttt gca ggc att 1590

Ser Gly Asp Ser Asn Pro Leu His Ile Asp Pro Ser Phe Ala Gly Ile
510 515 520

gcc ggt ttt gag aaa ccc ata tta cac gga tta tgt act ttt ggg ttt 1638
Ala Gly Phe Glu Lys Pro Ile Leu His Gly Leu Cys Thr Phe Gly Phe
525 530 535

tct gca agg cat gtt tta cag cag ttt gcg gat aat gat gtg tca aga 1686
Ser Ala Arg His Val Leu Gln Gln Phe Ala Asp Asn Asp Val Ser Arg
540 545 550

ttc aag gcc att aag gtt cgt ttt gcc aaa cca gtg tat cca gga caa 1734
Phe Lys Ala Ile Lys Val Arg Phe Ala Lys Pro Val Tyr Pro Gly Gln
555 560 565

act cta caa act gag atg tgg aag gaa gga aac aga att cat ttt caa 1782
Thr Leu Gln Thr Glu Met Trp Lys Glu Gly Asn Arg Ile His Phe Gln
570 575 580 585

acc aag gtc caa gag act gga gac att gtc att tcc aat gca tat gtg 1830
Thr Lys Val Gln Glu Thr Gly Asp Ile Val Ile Ser Asn Ala Tyr Val
590 595 600

gat ctt gtt cct aca tct gga gtt tcc gct cag aca cct tct gag ggt 1878
Asp Leu Val Pro Thr Ser Gly Val Ser Ala Gln Thr Pro Ser Glu Gly
605 610 615

gga gca ctg cag agt gct ctt gta ttt ggg gaa ata ggt cga cgc ctc 1926
Gly Ala Leu Gln Ser Ala Leu Val Phe Gly Glu Ile Gly Arg Arg Leu
620 625 630

aag gat gtt gga cgt gag gtg gta aag aaa gta aat gct gta ttt gaa 1974
Lys Asp Val Gly Arg Glu Val Val Lys Lys Val Asn Ala Val Phe Glu
635 640 645

tgg cat atc acg aaa aat ggg aat gtt gca gcc aag tgg acc att gac 2022
Trp His Ile Thr Lys Asn Gly Asn Val Ala Ala Lys Trp Thr Ile Asp
650 655 660 665

ctg aag aac ggc tct gga gag gtt tac caa ggc cct gcc aaa ggc tct 2070
Leu Lys Asn Gly Ser Gly Glu Val Tyr Gln Gly Pro Ala Lys Gly Ser
670 675 680

gct gac acg acc atc aca att tct gat gag gat ttc atg gaa gtg gtc 2118
Ala Asp Thr Thr Ile Thr Ile Ser Asp Glu Asp Phe Met Glu Val Val
685 690 695

ctg ggc aag ctt aac cca cag aat gcc ttc ttc agt ggc aga ctg aag 2166
Leu Gly Lys Leu Asn Pro Gln Asn Ala Phe Phe Ser Gly Arg Leu Lys
700 705 710

gcc cga gga aac atc atg ctg agc cag aag cta cag atg att ctg aaa 2214
Ala Arg Gly Asn Ile Met Leu Ser Gln Lys Leu Gln Met Ile Leu Lys
715 720 725

gac tat gcc aag ctc tga aggacccact gcgtgcttta ataaaaccag          2262
Asp Tyr Ala Lys Leu
730

aatcattacg ttctgtctac gcagtcatgc tccagccttc tttgaaacga tccacggtaa   2322


<210>  2
<211>  734
<212>  PRT
<213>  Rattus norvegicus

<400>  2

Met Ser Pro Leu Arg Phe Asp Gly Arg Val Gly Leu Val Thr Gly Ala
1               5                   10                  15

Gly Gly Gly Leu Gly Arg Ala Tyr Gly Leu Ala Phe Ala Glu Arg Gly
            20                  25                  30

Ala Leu Val Val Val Asn Asp Leu Gly Gly Asp Phe Lys Gly Val Gly
        35                  40                  45

Lys Gly Ser Ser Ala Ala Asp Lys Val Val Glu Glu Ile Arg Arg Arg
    50                  55                  60

Gly Gly Lys Ala Val Ala Asn Tyr Asp Ser Val Glu Ala Gly Glu Lys
65                  70                  75                  80

Leu Val Lys Thr Ala Leu Asp Thr Phe Gly Arg Ile Asp Val Val Val
                85                  90                  95

Asn Asn Ala Gly Ile Leu Arg Asp Pro Ser Phe Ser Arg Ile Ser Asp
            100                 105                 110

Glu Asp Trp Asp Ile Ile Gln Arg Val His Leu Arg Gly Ser Phe Gln
        115                 120                 125

Val Thr Arg Ala Ala Trp Asp His Met Lys Lys Gln Asn Tyr Gly Arg
    130                 135                 140

Ile Ile Met Thr Ala Ser Ala Ser Gly Ile Tyr Gly Asn Phe Gly Gln
145                 150                 155                 160

Ala Asn Tyr Ser Ala Ala Lys Leu Gly Leu Leu Gly Leu Ala Asn Thr
                165                 170                 175

Leu Val Ile Glu Gly Arg Lys Asn Asn Ile His Cys Asn Thr Ile Ala
            180                 185                 190

Pro Asn Ala Gly Ser Arg Met Thr Glu Thr Val Met Pro Glu Asp Leu
        195                 200                 205

Val Glu Ala Leu Lys Pro Glu Tyr Val Ala Pro Leu Val Leu Trp Leu
    210                 215                 220

Cys His Glu Ser Cys Glu Glu Asn Gly Gly Leu Phe Glu Val Gly Ala
225                 230                 235                 240

Gly Trp Ile Gly Lys Leu Arg Trp Glu Arg Thr Leu Gly Ala Ile Val
                245                 250                 255

Arg Lys Arg Asn Gln Pro Met Thr Pro Glu Ala Val Arg Asp Asn Trp
            260                 265                 270

Val Lys Ile Cys Asp Phe Ser Asn Ala Ser Glu Pro Lys Ser Ile Gln
        275                 280                 285

Glu Ser Thr Gly Gly Ile Ile Glu Val Leu His Lys Ile Asp Ser Glu
    290                 295                 300

Gly Ile Ser Gln Asn His Thr Gly Gln Val Ala Ser Ala Asp Ala Ser
305                 310                 315                 320

Gly Phe Ala Gly Val Val Gly His Lys Leu Pro Ser Phe Ser Ser Ser
                325                 330                 335

Tyr Thr Glu Leu Gln Cys Ile Met Tyr Ala Leu Gly Val Gly Ala Ser
            340                 345                 350

Val Lys Asn Pro Lys Asp Leu Lys Phe Val Tyr Glu Gly Ser Pro Asp
        355                 360                 365

Phe Ser Cys Leu Pro Thr Ile Gly Val Ile Val Ala Gln Lys Ser Leu
    370                 375                 380

Met Ser Gly Gly Leu Ala Glu Val Pro Gly Leu Ser Ile Asn Phe Ala
385                 390                 395                 400

Lys Val Leu His Gly Glu Gln Tyr Leu Glu Leu Tyr Lys Pro Leu Pro
                405                 410                 415

Arg Ser Gly Glu Leu Lys Cys Glu Ala Val Ile Ala Asp Ile Leu Asp
            420                 425                 430

Lys Gly Ser Gly Ile Val Ile Val Met Asp Val Tyr Ser Tyr Ser Gly
        435                 440                 445

Lys Glu Leu Ile Cys Tyr Asn Gln Phe Ser Val Phe Val Val Gly Ser
    450                 455                 460

Gly Gly Phe Gly Gly Lys Arg Thr Ser Glu Lys Leu Lys Ala Ala Val
465                 470                 475                 480

Ala Val Pro Ser Arg Pro Pro Asp Ala Val Leu Arg Asp Thr Thr Ser
                485                 490                 495

Val Asn Gln Ala Ala Leu Tyr Arg Leu Ser Gly Asp Ser Asn Pro Leu
            500                 505                 510

His Ile Asp Pro Ser Phe Ala Gly Ile Ala Gly Phe Glu Lys Pro Ile
        515                 520                 525

Leu His Gly Leu Cys Thr Phe Gly Phe Ser Ala Arg His Val Leu Gln
    530                 535                 540

Gln Phe Ala Asp Asn Asp Val Ser Arg Phe Lys Ala Ile Lys Val Arg
545                 550                 555                 560

Phe Ala Lys Pro Val Tyr Pro Gly Gln Thr Leu Gln Thr Glu Met Trp
                565                 570                 575

Lys Glu Gly Asn Arg Ile His Phe Gln Thr Lys Val Gln Glu Thr Gly
            580                 585                 590

Asp Ile Val Ile Ser Asn Ala Tyr Val Asp Leu Val Pro Thr Ser Gly
        595                 600                 605

Val Ser Ala Gln Thr Pro Ser Glu Gly Gly Ala Leu Gln Ser Ala Leu
    610                 615                 620

Val Phe Gly Glu Ile Gly Arg Arg Leu Lys Asp Val Gly Arg Glu Val
625                 630                 635                 640

Val Lys Lys Val Asn Ala Val Phe Glu Trp His Ile Thr Lys Asn Gly
                645                 650                 655

Asn Val Ala Ala Lys Trp Thr Ile Asp Leu Lys Asn Gly Ser Gly Glu
            660                 665                 670

Val Tyr Gln Gly Pro Ala Lys Gly Ser Ala Asp Thr Thr Ile Thr Ile
        675                 680                 685

Ser Asp Glu Asp Phe Met Glu Val Val Leu Gly Lys Leu Asn Pro Gln
    690                 695                 700

Asn Ala Phe Phe Ser Gly Arg Leu Lys Ala Arg Gly Asn Ile Met Leu
705                 710                 715                 720

Ser Gln Lys Leu Gln Met Ile Leu Lys Asp Tyr Ala Lys Leu
                725                 730

<210>  3
<211>  2593
<212>  DNA
<213>  Homo sapiens

<220>
<221>  CDS
<222>  (49)..(2259)
<223>

<400> 3
ggccagcgcg tctgcttgtt cgtgtgtgtg tcgttgcagg ccttattc atg ggc tca 57
ccg ctg agg ttc gac ggg cgg gtg gta ctg gtc acc ggc gcg ggg gca 105
gga ttg ggc cga gcc tat gcc ctg gct ttt gca gaa aga gga gcg tta 153
gtt gtt gtg aat gat ttg gga ggg gac ttc aaa gga gtt ggt aaa ggc 201
tcc tta gct gct gat aag gtt gtt gaa gaa ata aga agg aga ggt gga 249
aaa gca gtg gcc aac tat gat tca gtg gaa gaa gga gag aag gtt gtg 297
aag aca gcc ctg gat gct ttt gga aga ata gat gtt gtg gtc aac aat 345
gct gga att ctg agg gat cgt tcc ttt gct agg ata agt gat gaa gac 393
tgg gat ata atc cac aga gtt cat ttg cgg ggt tca ttc caa gtg aca 441
cgg gca gca tgg gaa cac atg aag aaa cag aag tat gga agg att att 489
atg act tca tca gct tca gga ata tat ggc aac ttt ggc cag gcc aat 537
tat agt gct gca aag ttg ggt ctt ctg ggc ctt gca aat tct ctt gca 585
att gaa ggc agg aaa agc aac att cat tgt aac acc att gct cct aat 633
gcg gga tca cgg atg act cag aca gtt atg cct gaa gat ctt gtg gaa     681
gcc ctg aag cca gag tat gtg gca cct ctt gtc ctt tgg ctt tgt cac     729
gag agt tgt gag gag aat ggt ggc ttg ttt gag gtt gga gca gga tgg     777
att gga aaa tta cgc tgg gag cgg act ctt gga gct att gta aga caa     825
aag aat cac cca atg act cct gag gca gtc aag gct aac tgg aag aag     873
atc tgt gac ttt gag aat gcc agc aag cct cag agt atc caa gaa tca     921
act ggc agt ata att gaa gtt ctg agt aaa ata gat tca gaa gga gga     969
gtt tca gca aat cat act agt cgt gca acg tct aca gca aca tca gga     1017
ttt gct gga gct att ggc cag aaa ctc cct cca ttt tct tat gct tat     1065
acg gaa ctg gaa gct att atg tat gcc ctt gga gtg gga gcg tca atc     1113
aag gat cca aaa gat ttg aaa ttt att tat gaa gga agt tct gat ttc     1161
tcc tgt ttg ccc acc ttc gga gtt atc ata ggt cag aaa tct atg atg     1209
ggt gga gga tta gca gaa att cct gga ctt tca atc aac ttt gca aag     1257
gtt ctt cat gga gag cag tac tta gag tta tat aaa cca ctt ccc aga     1305
gca gga aaa tta aaa tgt gaa gca gtt gtt gct gat gtc cta gat aaa      1353
gga tcc ggt gta gtg att att atg gat gtc tat tct tat tct gag aag      1401
gaa ctt ata tgc cac aat cag ttc tct ctc ttt ctt gtt ggc tct gga      1449
ggc ttt ggt gga aaa cgg aca tca gac aaa gtc aag gta gct gta gcc      1497
ata cct aat aga cct cct gat gct gta ctt aca gat acc acc tct ctt      1545
aat cag gct gct ttg tac cgc ctc agt gga gac tgg aat ccc tta cac      1593
att gat cct aac ttt gct agt cta gca ggt ttt gac aag ccc ata tta      1641
cat gga tta tgt aca ttt gga ttt tct gcc agg cgt gtg tta cag cag      1689
ttt gca gat aat gat gtg tca aga ttc aag gca att aag gct cgt ttt      1737
gca aaa cca gta tat cca gga caa act cta caa act gag atg tgg aag      1785
gaa gga aac aga att cat ttt caa acc aag gtc caa gaa act gga gac      1833
att gtc att tca aat gca tat gtg gat ctt gca cca aca tct ggt act      1881
tca gct aag aca ccc tct gag ggc ggg aag ctt cag agt acc ttt gta      1929
ttt gag gaa ata gga cgc cgc cta aag gat att ggg cct gag gtg gtg     1977
aag aaa gta aat gct gta ttt gag tgg cat ata acc aaa ggc gga aat     2025
att ggg gct aag tgg act att gac ctg aaa agt ggt tct gga aaa gtg     2073
tac caa ggc cct gca aaa ggt gct gct gat aca aca atc ata ctt tca     2121
gat gaa gat ttc atg gag gtg gtc ctg ggc aag ctt gac cct cag aag     2169
gca ttc ttt agt ggc agg ctg aag gcc aga ggg aac atc atg ctg agc     2217
cag aaa ctt cag atg att ctt aaa gac tac gcc aag ctc tga             2259
agggcacact acactattaa taaaaatgga atcattaaat actctcttca cccaaatatg   2319
cttgattatt ctgcaaaagt gattagaact aagatgcagg ggaaattgct taacattttc   2379
agatatcaga taactgcaga ttttcatttt ctactaattt tcatgtatca ttatttttac   2439
aaggaactat atataagcta gcacatgatt atccttctgt tcttagatct gtatcttcat   2499
aataaaaaat tttgcccaag tcctgtttcc ttagaatttg tgatagcatt gataagttga   2559
aaggaaaatt aaatcaataa aggcctttga tacc                               2593

<210>  4
<211>  736
<212>  PRT
<213>  Homo sapiens

<400>  4

<210>  5
<211>  2468
<212>  DNA
<213>  Mus musculus

<220>
<221>  CDS
<222>  (13)..(2220)
<223>

<400>  5

caggctgagc tc atg gct tcc ccc ctg agg ttc gac ggg cgt gtg gtc ttg      51
              Met Ala Ser Pro Leu Arg Phe Asp Gly Arg Val Val Leu
              1               5                   10

gtc acc ggc ccc ggg gga gga ttg ggc cga gct tac gcc ctg gcg ttt      99
Val Thr Gly Pro Gly Gly Gly Leu Gly Arg Ala Tyr Ala Leu Ala Phe
    15                  20                  25

gca gaa aga gga gca tta gtc att gtg aac gac tta gga ggg gac ttc      147
Ala Glu Arg Gly Ala Leu Val Ile Val Asn Asp Leu Gly Gly Asp Phe
30                  35                  40                  45

aag gga att ggt aaa ggc tcc tct gct gca gac aag gtt gtg gca gag      195
Lys Gly Ile Gly Lys Gly Ser Ser Ala Ala Asp Lys Val Val Ala Glu
                50                  55                  60

ata aga agg aaa ggc gga aaa gca gtg gcc aat tac gat tca gtt gaa      243
Ile Arg Arg Lys Gly Gly Lys Ala Val Ala Asn Tyr Asp Ser Val Glu
            65                  70                  75

gca ggc gag aag ctt gtg aag acg gca ctg gac aca ttt ggc aga ata      291
Ala Gly Glu Lys Leu Val Lys Thr Ala Leu Asp Thr Phe Gly Arg Ile
        80                  85                  90

gac gtt gtg gtc aac aat gct gga atc ctg agg gac cgt tcc ttc tcc 339
Asp Val Val Val Asn Asn Ala Gly Ile Leu Arg Asp Arg Ser Phe Ser
    95                  100                 105

agg ata agt gat gaa gac tgg gat ata att cat aga gtt cat ttg cgg 387
Arg Ile Ser Asp Glu Asp Trp Asp Ile Ile His Arg Val His Leu Arg
110                 115                 120                 125

ggc tcc ttc caa gtg acc cgg gca gca tgg gac cat atg aag aaa cag 435
Gly Ser Phe Gln Val Thr Arg Ala Ala Trp Asp His Met Lys Lys Gln
                130                 135                 140

aat tat gga aga atc ctt atg act tcc tca gct tct gga ata tat ggc 483
Asn Tyr Gly Arg Ile Leu Met Thr Ser Ser Ala Ser Gly Ile Tyr Gly
            145                 150                 155

aac ttt ggc cag gcg aat tat agt gct gca aag ctg ggc att ctg ggt 531
Asn Phe Gly Gln Ala Asn Tyr Ser Ala Ala Lys Leu Gly Ile Leu Gly
        160                 165                 170

ctc tgc aat act ctc gcc att gaa ggc agg aag aac aac att cat tgc 579
Leu Cys Asn Thr Leu Ala Ile Glu Gly Arg Lys Asn Asn Ile His Cys
    175                 180                 185

aac acc att gcc ccc aac gct ggg tca cgg atg acg gag act gtg ttg 627
Asn Thr Ile Ala Pro Asn Ala Gly Ser Arg Met Thr Glu Thr Val Leu
190                 195                 200                 205

ccg gaa gat ctt gtt gaa gcc ctg aag cca gag tat gtg gcc cct ctg 675
Pro Glu Asp Leu Val Glu Ala Leu Lys Pro Glu Tyr Val Ala Pro Leu
                210                 215                 220

gtg ctt tgg ctt tgc cat gag agc tgt gag gaa aat ggt ggc cta ttt 723
Val Leu Trp Leu Cys His Glu Ser Cys Glu Glu Asn Gly Gly Leu Phe
            225                 230                 235

gag gtt gga gca gga tgg att gga aaa ttg cgc tgg gag agg acc ctg 771
Glu Val Gly Ala Gly Trp Ile Gly Lys Leu Arg Trp Glu Arg Thr Leu
        240                 245                 250

ggc gcc atc gtc aga aag cgg aat cag ccc atg act ccc gag gca gtg 819
Gly Ala Ile Val Arg Lys Arg Asn Gln Pro Met Thr Pro Glu Ala Val
    255                 260                 265

agg gac aac tgg gag aag atc tgt gac ttc agc aat gcc agc aag ccg 867
Arg Asp Asn Trp Glu Lys Ile Cys Asp Phe Ser Asn Ala Ser Lys Pro
270                 275                 280                 285

cag acc att caa gaa tca aca ggt ggt ata gtc gaa gtt tta cat aag 915
Gln Thr Ile Gln Glu Ser Thr Gly Gly Ile Val Glu Val Leu His Lys
                290                 295                 300

gta gat tca gaa gga atc tca cca aac cgt acc agt cac gcg gca cct 963

Val Asp Ser Glu Gly Ile Ser Pro Asn Arg Thr Ser His Ala Ala Pro
            305                 310                 315

gca gcc acg tca gga ttc gtt ggt gct gtt ggc cat aaa ctt cct tca      1011
Ala Ala Thr Ser Gly Phe Val Gly Ala Val Gly His Lys Leu Pro Ser
        320                 325                 330

ttt tct tct tcg tat acg gag ctg cag agt att atg tat gcc ctc gga      1059
Phe Ser Ser Ser Tyr Thr Glu Leu Gln Ser Ile Met Tyr Ala Leu Gly
    335                 340                 345

gtg gga gcg tca gtc aaa aat cca aag gat ttg aag ttt gtt tat gaa      1107
Val Gly Ala Ser Val Lys Asn Pro Lys Asp Leu Lys Phe Val Tyr Glu
350                 355                 360                 365

ggc agt gct gac ttc tcc tgt ttg ccc acc ttc gga gtc att gtc gct      1155
Gly Ser Ala Asp Phe Ser Cys Leu Pro Thr Phe Gly Val Ile Val Ala
                370                 375                 380

cag aag tcc atg atg aat gga ggg ctg gca gag gtt cct ggg ctg tca      1203
Gln Lys Ser Met Met Asn Gly Gly Leu Ala Glu Val Pro Gly Leu Ser
            385                 390                 395

ttc aac ttt gca aag gct ctt cac ggg gag cag tac ttg gag ctg tat      1251
Phe Asn Phe Ala Lys Ala Leu His Gly Glu Gln Tyr Leu Glu Leu Tyr
        400                 405                 410

aag cca ctt ctt cga tca gga gaa tta aaa tgt gaa gca gtt att gct      1299
Lys Pro Leu Leu Arg Ser Gly Glu Leu Lys Cys Glu Ala Val Ile Ala
    415                 420                 425

gac atc ctg gat aaa ggc tct ggc gta gtg att gtt atg gac gtc tat      1347
Asp Ile Leu Asp Lys Gly Ser Gly Val Val Ile Val Met Asp Val Tyr
430                 435                 440                 445

tct tat tct ggg aag gaa ctt ata tgc tat aat cag ttc tct gtc ttt      1395
Ser Tyr Ser Gly Lys Glu Leu Ile Cys Tyr Asn Gln Phe Ser Val Phe
                450                 455                 460

gtt gtt ggc tct ggg ggc ttt ggt gga aaa cgg aca tca gaa aaa ctc      1443
Val Val Gly Ser Gly Gly Phe Gly Gly Lys Arg Thr Ser Glu Lys Leu
            465                 470                 475

aaa gca gct gta gct gta cca aat cga cct cca gat gct gta ctg aga      1491
Lys Ala Ala Val Ala Val Pro Asn Arg Pro Pro Asp Ala Val Leu Arg
        480                 485                 490

gat gcc acc tca ctg aat cag gcc gcg ctg tac cgc ctc agc gga gac      1539
Asp Ala Thr Ser Leu Asn Gln Ala Ala Leu Tyr Arg Leu Ser Gly Asp
    495                 500                 505

tgg aat cct cta cac att gac ccg gac ttt gcg agc gtt gcc ggt ttt      1587
Trp Asn Pro Leu His Ile Asp Pro Asp Phe Ala Ser Val Ala Gly Phe
510                 515                 520                 525

gag aag ccc ata tta cat gga cta tgt acc ttt gga ttt tct gca agg 1635
Glu Lys Pro Ile Leu His Gly Leu Cys Thr Phe Gly Phe Ser Ala Arg
                530                 535                 540

cat gtt tta cag cag ttt gca gat aat gat gta tca aga ttc aag gcg 1683
His Val Leu Gln Gln Phe Ala Asp Asn Asp Val Ser Arg Phe Lys Ala
            545                 550                 555

att aag gtt cgt ttt gcc aaa cca gtg tat cca gga cag act cta caa 1731
Ile Lys Val Arg Phe Ala Lys Pro Val Tyr Pro Gly Gln Thr Leu Gln
        560                 565                 570

act gag atg tgg aag gaa gga aac aga att cat ttt caa acc aag gtc 1779
Thr Glu Met Trp Lys Glu Gly Asn Arg Ile His Phe Gln Thr Lys Val
    575                 580                 585

cac gag act gga gat gtt gtc att tca aat gcg tac gtg gat ctc gtg 1827
His Glu Thr Gly Asp Val Val Ile Ser Asn Ala Tyr Val Asp Leu Val
590                 595                 600                 605

cct gca tct gga gtt tca acc cag aca cct tca gag ggt gga gag ctc 1875
Pro Ala Ser Gly Val Ser Thr Gln Thr Pro Ser Glu Gly Gly Glu Leu
                610                 615                 620

cag agt gct ctt gtg ttt ggg gag ata ggc cgc cgc ctc aag agt gtt 1923
Gln Ser Ala Leu Val Phe Gly Glu Ile Gly Arg Arg Leu Lys Ser Val
            625                 630                 635

ggc cgt gag gtg gta aag aaa gcg aat gct gtg ttt gaa tgg cat atc 1971
Gly Arg Glu Val Val Lys Lys Ala Asn Ala Val Phe Glu Trp His Ile
        640                 645                 650

acg aaa ggt ggg act gtt gca gcc aag tgg acc att gac ctg aag agc 2019
Thr Lys Gly Gly Thr Val Ala Ala Lys Trp Thr Ile Asp Leu Lys Ser
    655                 660                 665

ggc tca ggg gag gtg tac caa ggc ccc gca aag ggc tct gct gat gtg 2067
Gly Ser Gly Glu Val Tyr Gln Gly Pro Ala Lys Gly Ser Ala Asp Val
670                 675                 680                 685

acc atc atc att tcc gat gag gat ttt atg gaa gtg gtc ttc ggc aag 2115
Thr Ile Ile Ile Ser Asp Glu Asp Phe Met Glu Val Val Phe Gly Lys
                690                 695                 700

ctt gac cca cag aag gcc ttc ttc agt ggc agg ctg aag gcc aga ggg 2163
Leu Asp Pro Gln Lys Ala Phe Phe Ser Gly Arg Leu Lys Ala Arg Gly
            705                 710                 715

aac atc atg ctg agc cag aaa cta cag atg att ctt aaa gac tat gcc 2211
Asn Ile Met Leu Ser Gln Lys Leu Gln Met Ile Leu Lys Asp Tyr Ala
        720                 725                 730

aag ctc tga agggaaccca ctgtgtgctg ttaaaggagt caataattaa 2260

Lys Leu
    735

atactgtcta cccagctgag ccgcagcctt ctgcgatcca caggagtgtg caggagaaat    2320
cgcttcacat ttccagattc agataacttg catattttca ttttctacta atttttcaca    2380
tatttttaca aggaactgta atctaggtag caaaataatc attctgttca tagatctgta    2440
tcttaataaa aaaaatcaac caaaaacc                                        2468

<210>  6
<211>  735
<212>  PRT
<213>  Mus musculus

<400>  6

Met Ala Ser Pro Leu Arg Phe Asp Gly Arg Val Val Leu Val Thr Gly
1               5                   10                  15

Pro Gly Gly Gly Leu Gly Arg Ala Tyr Ala Leu Ala Phe Ala Glu Arg
            20                  25                  30

Gly Ala Leu Val Ile Val Asn Asp Leu Gly Gly Asp Phe Lys Gly Ile
        35                  40                  45

Gly Lys Gly Ser Ser Ala Ala Asp Lys Val Val Ala Glu Ile Arg Arg
    50                  55                  60

Lys Gly Gly Lys Ala Val Ala Asn Tyr Asp Ser Val Glu Ala Gly Glu
65                  70                  75                  80

Lys Leu Val Lys Thr Ala Leu Asp Thr Phe Gly Arg Ile Asp Val Val
                85                  90                  95

Val Asn Asn Ala Gly Ile Leu Arg Asp Arg Ser Phe Ser Arg Ile Ser
            100                 105                 110

Asp Glu Asp Trp Asp Ile Ile His Arg Val His Leu Arg Gly Ser Phe
        115                 120                 125

Gln Val Thr Arg Ala Ala Trp Asp His Met Lys Lys Gln Asn Tyr Gly
    130                 135                 140

Arg Ile Leu Met Thr Ser Ser Ala Ser Gly Ile Tyr Gly Asn Phe Gly
145                 150                 155                 160

Gln Ala Asn Tyr Ser Ala Ala Lys Leu Gly Ile Leu Gly Leu Cys Asn
                165                 170                 175

Thr Leu Ala Ile Glu Gly Arg Lys Asn Asn Ile His Cys Asn Thr Ile
            180                 185                 190

Ala Pro Asn Ala Gly Ser Arg Met Thr Glu Thr Val Leu Pro Glu Asp
        195                 200                 205

Leu Val Glu Ala Leu Lys Pro Glu Tyr Val Ala Pro Leu Val Leu Trp
    210                 215                 220

Leu Cys His Glu Ser Cys Glu Glu Asn Gly Gly Leu Phe Glu Val Gly
225                 230                 235                 240

Ala Gly Trp Ile Gly Lys Leu Arg Trp Glu Arg Thr Leu Gly Ala Ile
                245                 250                 255

Val Arg Lys Arg Asn Gln Pro Met Thr Pro Glu Ala Val Arg Asp Asn
            260                 265                 270

Trp Glu Lys Ile Cys Asp Phe Ser Asn Ala Ser Lys Pro Gln Thr Ile
        275                 280                 285

Gln Glu Ser Thr Gly Gly Ile Val Glu Val Leu His Lys Val Asp Ser
    290                 295                 300

Glu Gly Ile Ser Pro Asn Arg Thr Ser His Ala Ala Pro Ala Ala Thr
305                 310                 315                 320

Ser Gly Phe Val Gly Ala Val Gly His Lys Leu Pro Ser Phe Ser Ser
                325                 330                 335

Ser Tyr Thr Glu Leu Gln Ser Ile Met Tyr Ala Leu Gly Val Gly Ala
            340                 345                 350

Ser Val Lys Asn Pro Lys Asp Leu Lys Phe Val Tyr Glu Gly Ser Ala
        355                 360                 365

Asp Phe Ser Cys Leu Pro Thr Phe Gly Val Ile Val Ala Gln Lys Ser
    370                 375                 380

Met Met Asn Gly Gly Leu Ala Glu Val Pro Gly Leu Ser Phe Asn Phe
385                 390                 395                 400

Ala Lys Ala Leu His Gly Glu Gln Tyr Leu Glu Leu Tyr Lys Pro Leu
                405                 410                 415

Leu Arg Ser Gly Glu Leu Lys Cys Glu Ala Val Ile Ala Asp Ile Leu
            420                 425                 430

Asp Lys Gly Ser Gly Val Val Ile Val Met Asp Val Tyr Ser Tyr Ser
        435                 440                 445

Gly Lys Glu Leu Ile Cys Tyr Asn Gln Phe Ser Val Phe Val Val Gly
    450                 455                 460

Ser Gly Gly Phe Gly Gly Lys Arg Thr Ser Glu Lys Leu Lys Ala Ala
465                 470                 475                 480

Val Ala Val Pro Asn Arg Pro Pro Asp Ala Val Leu Arg Asp Ala Thr
                485                 490                 495

Ser Leu Asn Gln Ala Ala Leu Tyr Arg Leu Ser Gly Asp Trp Asn Pro
                500                 505                 510

Leu His Ile Asp Pro Asp Phe Ala Ser Val Ala Gly Phe Glu Lys Pro
        515                 520                 525

Ile Leu His Gly Leu Cys Thr Phe Gly Phe Ser Ala Arg His Val Leu
    530                 535                 540

Gln Gln Phe Ala Asp Asn Asp Val Ser Arg Phe Lys Ala Ile Lys Val
545                 550                 555                 560

Arg Phe Ala Lys Pro Val Tyr Pro Gly Gln Thr Leu Gln Thr Glu Met
                565                 570                 575

Trp Lys Glu Gly Asn Arg Ile His Phe Gln Thr Lys Val His Glu Thr
                580                 585                 590

Gly Asp Val Val Ile Ser Asn Ala Tyr Val Asp Leu Val Pro Ala Ser
        595                 600                 605

Gly Val Ser Thr Gln Thr Pro Ser Glu Gly Gly Glu Leu Gln Ser Ala
    610                 615                 620

Leu Val Phe Gly Glu Ile Gly Arg Arg Leu Lys Ser Val Gly Arg Glu
625                 630                 635                 640

Val Val Lys Lys Ala Asn Ala Val Phe Glu Trp His Ile Thr Lys Gly
                645                 650                 655

Gly Thr Val Ala Ala Lys Trp Thr Ile Asp Leu Lys Ser Gly Ser Gly
                660                 665                 670

Glu Val Tyr Gln Gly Pro Ala Lys Gly Ser Ala Asp Val Thr Ile Ile
        675                 680                 685

Ile Ser Asp Glu Asp Phe Met Glu Val Val Phe Gly Lys Leu Asp Pro
    690                 695                 700

Gln Lys Ala Phe Phe Ser Gly Arg Leu Lys Ala Arg Gly Asn Ile Met
705                 710                 715                 720

Leu Ser Gln Lys Leu Gln Met Ile Leu Lys Asp Tyr Ala Lys Leu
                725                 730                 735

## Description

### Technical Field

The present invention relates to a novel drug discovery target. More specifically, the present invention relates to a target molecule considered to be responsible for the efficacy of an anti-inflammatory agent or an anti-allergic agent, such as tiaramide, Intal and derivatives thereof. The present invention also relates to a compound that specifically binds to the target molecule.

### Background Art

With the decoding of the base sequences of the human genomes, subjects of research have been shifting to genomic drug discovery and search and identification of drug discovery targets. As such, subjects include the identification of targets considered to be responsible for the efficacy of tiaramide and derivatives thereof, or Intal and derivatives thereof, which have been commonly used as anti-inflammatory and anti-allergic agents.

To date, as a mechanism for tiaramide, which is classified as a non-acidic (basic) NSAIDs (non-steroidal anti-inflammatory drugs), an action to suppress the function of the prophlogistic factor histamine or serotonin at inflammatory sites and hence to suppress airway constriction has mainly been proposed (Chiryoyaku Manual 2004, p76; Arzneimittelforschung, 1972, Apr 22 (4), pp.724-732), and the suppression of TXA2 production has been shown to be a mechanism for the airway constriction suppression (see, for example, Folco GC et al., Arzneimittelforschung, Germany, 1982, 32(9), pp.1092-1095).

Also, Intal, known as an anti-allergic drug, is known to have suppressant action of the release of chemical mediators such as histamine and SRS-A from mast cells that occurs in relation to antigen-antibody reactions ( Chiryoyaku Manual 2004, p301), and the like; to date, regarding the action mechanisms thereof, available reports include the inhibition of the secretion of histamine and the like with phospholipase A stimulation (see, for example, Orr TS. and Cox JS, Nature, UK, 1969, 223 (202), pp.197-198), as well as the involvement of a protein involved in the calcium channel (see, for example, Mazurek N. et al., Proceedings of the National Academy of Sciences USA., USA, 1984, 81 (21), pp.6841-6845) and the involvement of intracellular protein kinase (see, for example, Theoharides TC. et al., Science, USA, 1980, 207 (4426), pp.80-82) and the like.

However, despite enormous efforts, identification of targets with which these drugs interact directly, and whose pharmacological activity be explained, has been an unresolved problem. For this reason, and also because of difficulty in developing an efficient method of screening for compounds surpassing these compounds in terms of efficacy, it has been extremely difficult to create a drug surpassing these compounds.

Whereas ordinary NSAIDs exhibit cyclooxygenase inhibition as their essential anti-inflammatory action, elucidation of this different type of effective anti-inflammatory mechanism is expected to lead to the discovery of a drug having high therapeutic effect that supplements the therapeutic effects of existing drugs such as cyclooxygenase inhibitors, which produce severe adverse reactions such as gastrointestinal disorder, or that replace them; therefore, elucidation of the true mechanism has been awaited. For anti-allergic agents as well, the same elucidation has been awaited.

It is an object of the present invention to identify a target considered to be responsible for the efficacy of tiaramide and derivatives thereof, and Intal and derivatives thereof, as anti-inflammatory and anti-allergic agents, and to provide a screening method for a compound useful in the treatment of a disease such as an inflammatory disease or an allergic disease using the target, and a compound obtained by the screening.

### Disclosure of the Invention

The present inventors diligently investigated to solve the above-described problems in search of a protein to which Intal and tiaramide bind specifically in common. As a result, the present inventors found that a protein called MFP-2 (multifunctional protein 2; Accession No. P70523) specifically binds to Intal and tiaramide, confirmed that the protein is a sufficient drug discovery target to explain the anti-inflammatory and anti-allergic actions of these derivatives, developed a screening method for a compound useful in the treatment of an inflammatory disease or an allergic disease using such a target or cells expressing the target, and thus developed the present invention.

Accordingly, the present invention relates to the following:
[1] A pharmaceutical composition comprising as an active ingredient a compound that specifically binds to a protein having the amino acid sequence of SEQ ID NO:2.
[2] A pharmaceutical composition comprising, as an active ingredient, a compound that specifically binds to a protein, which is characterized by having an amino acid sequence resulting from the deletion, substitution or addition of one or more amino acids in the amino acid sequence of SEQ ID NO:2 and binding to a compound of the formula (1) and/or a compound of the formula (2). 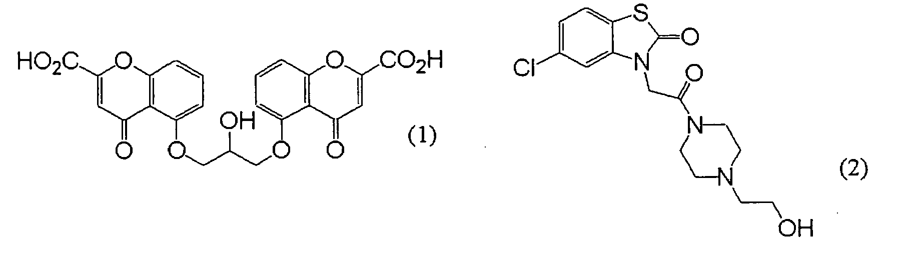
[3] The pharmaceutical composition described in [1] or [2] above, which is for the treatment of a disease selected from the group consisting of an inflammatory disease and an allergic disease.
[4] A pharmaceutical composition comprising as an active ingredient a compound that specifically binds to MFP-2.
[5] A pharmaceutical composition comprising as an active ingredient a compound that regulates the expression of MFP-2.
[6] A pharmaceutical composition comprising as an active ingredient a compound that regulates the activity of MFP-2.
[7] The pharmaceutical composition described in any of [4] to [6] above, which is for the treatment of a disease selected from the group consisting of an inflammatory disease and an allergic disease.
[8] A method for screening for a compound useful for the treatment of a disease selected from the group consisting of an inflammatory disease and an allergic disease, which comprises the following steps of
   (1) bringing MFP-2 or a functional fragment thereof into contact with a test compound,
   (2) determining whether or not the test compound specifically binds to MFP-2 or a functional fragment thereof, and
   (3) selecting a test compound that specifically binds to MFP-2 or a functional fragment thereof in the step (2) above.
[9] A method for screening for a compound useful for the treatment of a disease selected from the group consisting of an inflammatory disease and an allergic disease, which comprises the following steps of
   (1) bringing a protein having the amino acid sequence of SEQ ID NO:2 or a functional fragment thereof into contact with a test compound,
   (2) determining whether or not the test compound specifically binds to the protein or a functional fragment thereof, and
   (3) selecting a test compound that specifically binds to the protein or a functional fragment thereof in the step (2) above.
[10] A method for screening for a compound useful for the treatment of a disease selected from the group consisting of an inflammatory disease and an allergic disease, which comprises the following steps of
   (1) bringing a protein, which is characterized by having an amino acid sequence resulting from the deletion, substitution or addition of one or more amino acids in the amino acid sequence of SEQ ID NO:2 and binding to a compound of the formula (1) and/or a compound of the formula (2), or a functional fragment thereof, into contact with a test compound, 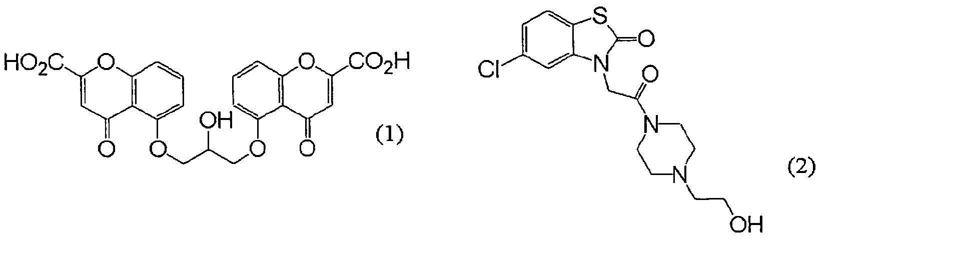
   (2) determining whether or not the test compound specifically binds to the protein or a functional fragment thereof, and
   (3) selecting a test compound that specifically binds to the protein or a functional fragment thereof in the step (2) above.
[11] A compound useful for the treatment of a disease selected from the group consisting of an inflammatory disease and an allergic disease, obtained by the screening method described in any of [8] to [10] above.
[12] A compound represented by the general formula (I) or the general formula (II) or a pharmaceutically acceptable salt thereof: 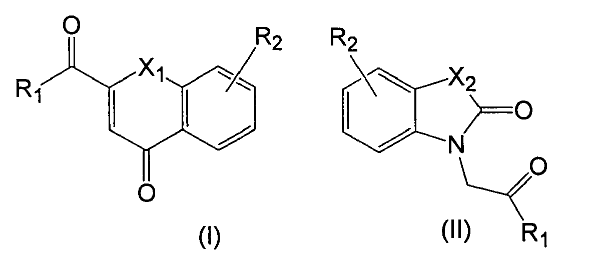 (wherein X₁ is O, NH, NR'", S, SO or SO₂;
   X₂ is O, NH, NR'", S, -CH₂CH₂- or -CH=CH-;
   R₁ is H, OH, OR', NH₂, NHR' or NR'R";
   R₂ is an optionally substituted saturated or unsaturated chain-structured hydrocarbon group, an optionally substituted alkoxy group, an optionally substituted amino group, an optionally substituted carboxyl group, an optionally substituted amide group or a halogen atom;
   R', R" and R'" are the same or different and each is an optionally substituted saturated or unsaturated chain-structured hydrocarbon group;
   provided that R₁ is NR'R", R' and R" may bind together to form a ring in cooperation with the adjoining nitrogen atom), but the compounds represented by the formula (1) and the formula (2) are excluded. 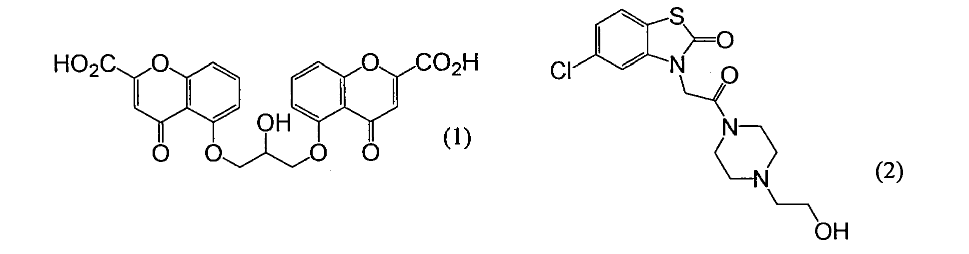
[13] A pharmaceutical composition comprising as an active ingredient the compound described in [12] above or a pharmaceutically acceptable salt thereof.
[14] The pharmaceutical composition described in [13] above, which is for the treatment of a disease selected from the group consisting of an inflammatory disease and an allergic disease.

### Brief Description of the Drawings

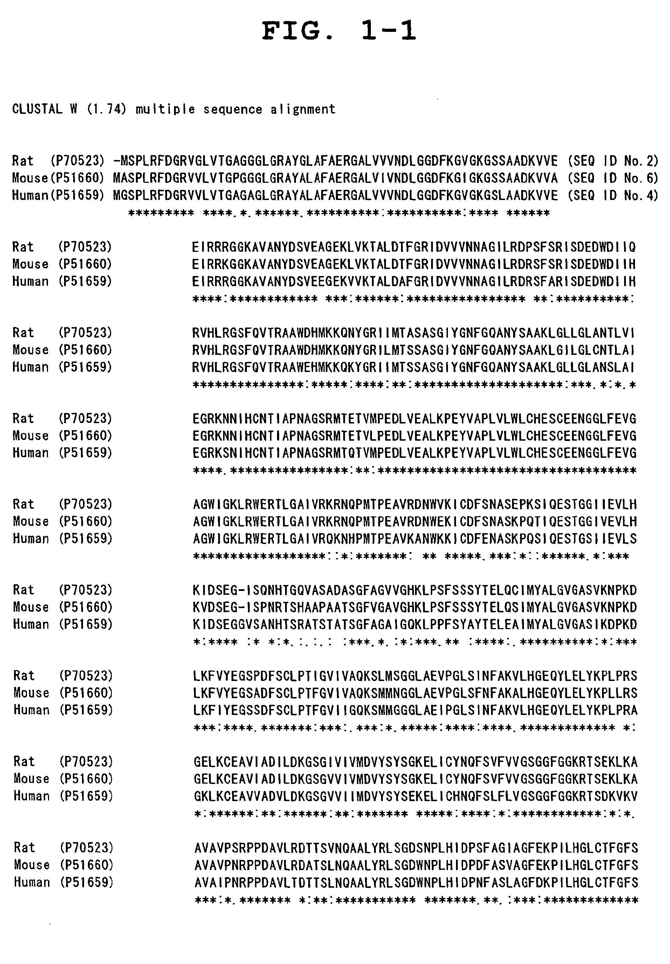

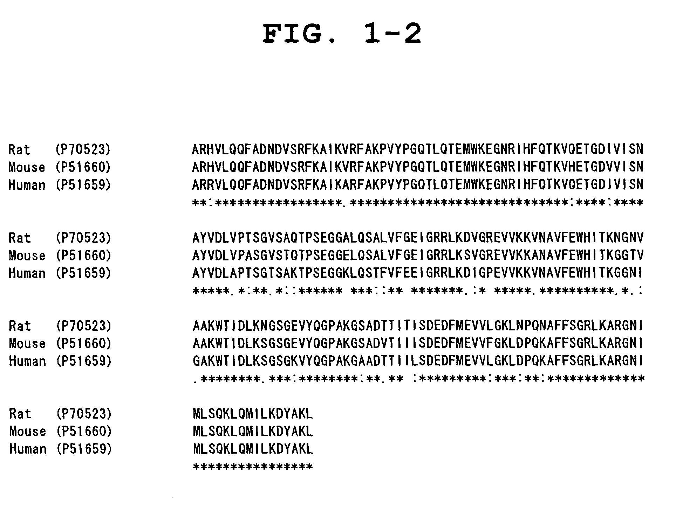

Figure 1-1 is a drawing showing the degrees of homology of MFP-2 among rats, mice, and humans.
Figure 1-2 is a drawing showing the degrees of homology of MFP-2 among rats, mice, and humans (continued from Figure 1-1).
Figure 2 is a drawing showing the results of an examination of the specificity of the MFP-2 binding of each compound. Onto a solid phase carrier with a compound that specifically binds to the MFP-2 immobilized thereon, MFP-2 binds quickly with a first immobilizing resin treatment.

### Detailed Description of the Invention

MFP-2 (multifunctional protein 2) has also been reported as rat-derived proteins such as multifunctional beta-oxidant protein 2, peroxisomal (Accession No. P70523), peroxisomal multifunctional enzyme type 2 (MFE-2), D-bifunctional protein (DBP), and 17-beta-hydroxysteroid dehydrogenase 4 (17-beta-HSD4) (all Accession No. P97852). In mice, the same protein has been reported as hydroxysteroid 17-beta dehydrogenase (Accession No. Q9DBM3), MFE-2, DBP, 17-beta-HSD4 (all Accession No. P51660) and the like; as human-derived proteins, the same protein has been reported as MFE-2, DBP, 17-beta-HSD4 (all Accession No. P51659) and the like. MFP-2 is a protein having a number of unlimited various synonyms. Structurally, MFP-2 is speculated to have many physiological actions because it has a short-chain dehydrogenase/reductase domain in the vicinity of the N-terminus thereof and a MaoC-like dehydratase domain and a sterol-binding domain-like domain on the C-terminus side. For example, MFP-2 is known to exhibit 2-enoyl-CoA hydratase and D-3-hydroxyacyl-CoA dehydrogenase activities, and to be involved in the beta oxidation of fatty acids.

More specifically, a protein comprising 734 amino acids, shown by the amino acid sequence of SEQ ID NO:2 (Accession No. P70523), can be mentioned as an example; as long as it binds specifically to Intal, tiaramide, derivatives thereof, or to a compound exhibiting anti-inflammatory action or anti-allergic action by a mechanism similar to that for these compounds, that is, acts as a target molecule, MFP-2 may be a protein having an amino acid sequence resulting from the deletion, substitution or addition of one or more amino acids in the amino acid sequence of SEQ ID NO:2. Therefore, although MFP-2 exhibits somewhat different amino acid sequences among species (see Figure 1), the MFP-2 of any species is an MFP-2 that can be used in the present invention. For example, the amino acid sequence of SEQ ID NO:2 is derived from the rat, but human MFP-2, which is shown by the amino acid sequence of SEQ ID NO:4, is also encompassed in the MFP-2 of the invention of this application.

More specifically, the MFP-2 that can be used in the present invention does not always need to be shown only by the amino acid sequence of SEQ ID NO:2, as long as it is a protein, which is characterized by binding to a compound of the formula (1) and/or a compound of the formula (2), 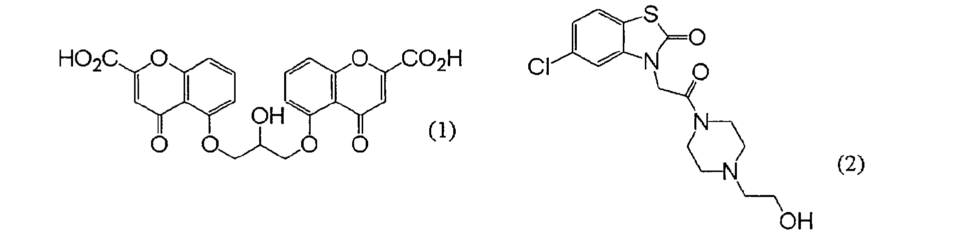 and it may be a protein having the amino acid sequence of SEQ ID NO:2, or a protein having an amino acid sequence resulting from the deletion, substitution or addition of one or more amino acids in the amino acid sequence of SEQ ID NO:2. More specifically, the MFP-2 is a protein shown by an amino acid sequence having a homology of 60% or more, 70% or more, 80% or more, preferably 90% or more, and more preferably 95% or more, to the amino acid sequence of SEQ ID NO:2, and is further preferably a protein (polypeptide) comprising 40 continuous amino acids or more, preferably 70 continuous amino acids or more, and particularly preferably 100 continuous amino acids or more, in the amino acid sequence of SEQ ID NO:2.

As used herein, "homology" means the degree of sequence correlation between two polypeptide sequences. Homology can easily be calculated. A large number of methods of measuring the homology between two polypeptide sequences are known, and the term "homology" (also called "identity") is obvious to those skilled in the art. Ordinary methods used to measure the homology of two sequences include, but are not limited to, those disclosed in Martin, J. Bishop (Ed.), Guide to Huge Computers, Academic Press, San Diego (1994); Carillo, H. & Lipman, D., SIAM J. Applied Math., 48:1073 (1988) and the like. As a preferable method for measuring the homology, one designed to obtain the largest matching portion between the two sequences tested can be mentioned. As such a method, one assembled in a computer program can be mentioned. Preferable computer programming methods for measuring the homology between two sequences include, but are not limited to, the GCG program package (Devereux, J. et al., Nucleic Acids Research, 12(1):387 (1984)), BLASTP, FASTA and the like; methods known in the art can be used.

Furthermore, in the present invention, the MFP-2 may be a fragment of MFP-2, as long as it is characterized by binding to a compound of the formula (1) and/or a compound of the formula (2), provided that it can serve in common as a target for a series of anti-inflammatory agents and anti-allergic agents such as Intal and tiaramide; such a fragment is hereinafter also referred to as a functional fragment of MFP-2. To increase the accuracy, it is desirable that the MFP-2 should bind to both a compound of the formula (1) and a compound of the formula (2).

All these embodiments are included in the MFP-2 in the present invention unless otherwise specified.

To determine whether or not the MFP-2 or a functional fragment thereof can be used in the present invention, a compound of the formula (1) and/or a compound of the formula (2), preferably both a compound of the formula (1) and a compound of the formula (2), are used; these compounds are already known compounds, and are commercially available or can be produced according to a known technology.

"specifically bind" is exemplified by the relation of a specific receptor to an agonist or an antagonist, the relation of an enzyme to a substrate, and the relation of, for example, an FK506-binding protein (target molecule) to FK506 (ligand), a steroid hormone receptor to a steroid hormone (e.g., dexamethason and glucocorticoid receptor), HDAC to the anticancer agent trapoxin, and the like, and can be confirmed as numerical values of Kd, Ka and the like by competitive experiments and the like. As described in Examples below, this can also be confirmed by a visual means such as electrophoresis, in addition to representation as specific numerical values.

The present invention provides a pharmaceutical composition comprising, as an active ingredient, a compound that specifically binds to MFP-2 (a protein having the amino acid sequence of SEQ ID NO:2, a protein, which is characterized by having the amino acid sequence of SEQ ID NO:2 and binding to a compound of the formula (1) and/or a compound of the formula (2), and the like) and a functional fragment thereof. Such a compound exhibits anti-inflammatory action and/or anti-allergic action by binding to MFP-2, which is a novel target for an anti-inflammatory agent or an anti-allergic agent, to regulate the expression of MFP-2 and/or regulate the activity thereof. An "inflammatory disease" is an exogenous or endogenous acute or chronic disease; in the case of acute disease, the five major signs of fever, reddening, swelling, pain and functional impairment are evident. For example, pain and inflammation following surgery in various departments and those following trauma, or pain and inflammation in arthritis, lumbago, cervico-omo-brachial syndrome, intrapelvic inflammation, damage of the soft birth canal, breast engorgement, herpes zoster, erythema exsudativum multiforme, cystitis, epididymitis, anterior ocular inflammation, pericoronitis of wisdom tooth, or after tooth extraction and the like, or diseases such as acute upper airway inflammation can be mentioned. As examples of the "allergic disease", diseases such as bronchial asthma, allergic rhinitis, and atopic dermatitis can be mentioned.

Furthermore, according to the finding obtained in the present invention that MFP-2 can serve as a novel drug discovery target for anti-inflammatory agents, anti-allergic agents and the like, a compound that does not bind directly to MFP-2 but does act directly or indirectly thereon to regulate the expression or activity of MFP-2 can also be said to be useful for the treatment of inflammatory disease and allergic disease.

As examples of the compound (substance) that regulates the expression or activity of MFP-2, a DNA that encodes MFP-2, a vector incorporating a DNA that encodes MFP-2, MFP-2 protein and the like can be mentioned.

A compound capable of binding to MFP-2 or a functional fragment thereof, like Intal or tiaramide, exhibits excellent anti-inflammatory action and/or anti-allergic action on various mammals, and is therefore useful as a therapeutic agent for inflammatory disease such as an anti-inflammatory agent, and as a therapeutic agent for allergic disease such as an anti-allergic agent (subject diseases are the same as described above).

As examples of the compound contained in the present invention as an active ingredient, specifically, a compound represented by the general formula (I) or the general formula (II) or a pharmaceutically acceptable salt thereof can be mentioned. 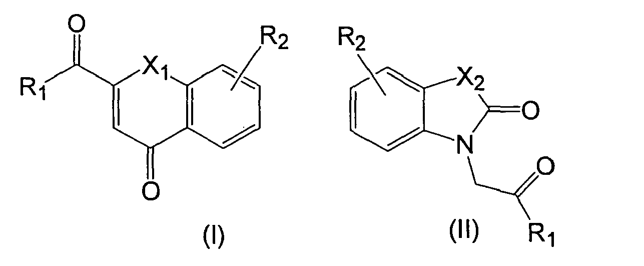 (wherein X₁ is O, NH, NR'", S, SO or SO₂;
X₂ is O, NH, NR'", S, -CH₂CH₂- or -CH=CH-;
R₁ is H, OH, OR', NH₂, NHR' or NR'R";
R₂ is an optionally substituted saturated or unsaturated chain-structured hydrocarbon group, an optionally substituted alkoxy group, an optionally substituted amino group, an optionally substituted carboxyl group, an optionally substituted amide group or a halogen atom;
R', R" and R'" are the same or different and each is an optionally substituted saturated or unsaturated chain-structured hydrocarbon group;
provided that R₁ is NR'R", R' and R" may bind together to form a ring in cooperation with the adjoining nitrogen atom), but the compounds represented by the formula (1) and the formula (2) are excluded.

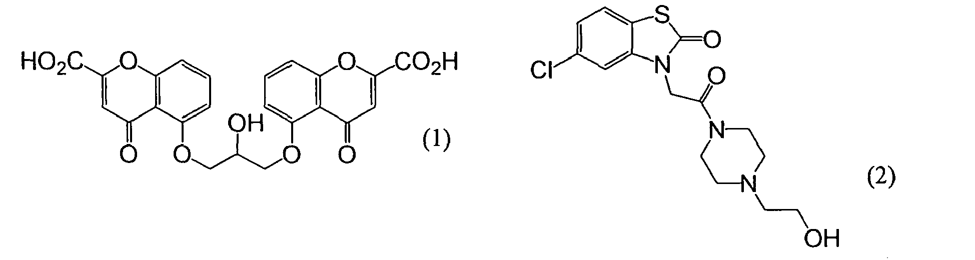

"A saturated or unsaturated chain-structured hydrocarbon group" denotes, for example, a linear or branched chain-structured hydrocarbon group having 1 to 10 carbon atoms, and the like; specifically, for example, an alkyl group, an alkenyl group, an alkynyl group and the like can be mentioned. Of these groups, an alkyl group is particularly preferable. As examples of the "alkyl group", an alkyl group having 1 to 10 carbon atoms, such as methyl, ethyl, n-propyl, isopropyl, butyl, isobutyl, sec-butyl, tert-butyl, n-pentyl, isopentyl, neopentyl, n-hexyl, and isohexyl, and the like can be mentioned. As examples of the "alkenyl group", an alkenyl group having 2 to 10 carbon atoms, such as vinyl, 1-propenyl, allyl, isopropenyl, 1-butenyl, 2-butenyl, 3-butenyl, isobutenyl, and sec-butenyl, and the like can be mentioned. As examples of the "alkynyl group", an alkynyl group having 2 to 10 carbon atoms, such as ethynyl, 1-propynyl, and propargyl, and the like can be mentioned.

The substituent for "an optionally substituted saturated or unsaturated chain-structured hydrocarbon group" is not subject to limitation; for example, a saturated or unsaturated cyclic hydrocarbon group (described below), a saturated or unsaturated heterocyclic group (described below), a halogen atom (described below), a cyano group, a nitro group, a hydroxyl group, an optionally substituted carboxyl group (described below), a substituted amide group (described below), an optionally substituted lower alkyl group (described below), an optionally substituted aroxy group, an optionally substituted amino group (described below), an optionally substituted alkoxy group and the like can be mentioned. These substituents substitute on the chain-structured hydrocarbon group, as long as the substitution is chemically acceptable. However, provided that the number of substituents is two or more, they may be the same or different.

"An alkoxy group" means a linear or branched alkoxy group having 1 to 6 carbon atoms; specifically, methoxy group, ethoxy group, n-propoxy group, isopropoxy group, n-butoxy group, isobutoxy group, sec-butoxy group, tert-butoxy group, n-pentyloxy group, isopentyloxy group, tert-pentyloxy group, neopentyloxy group, 2-pentyloxy group, 3-pentyloxy group, n-hexyloxy group, 2-hexyloxy group and the like can be mentioned.

The substituent for "an optionally substituted alkoxy group" is not subject to limitation; for example, a saturated or unsaturated cyclic hydrocarbon group (described below), a saturated or unsaturated heterocyclic group (described below), a halogen atom (described below), a cyano group, a nitro group, a hydroxyl group, an optionally substituted carboxyl group (described below), a substituted amide group (described below), an optionally substituted lower alkyl group (described below), an optionally substituted aroxy group, an optionally substituted amino group (described below), an optionally substituted alkoxy group and the like can be mentioned. These substituents substitute on the alkoxy group, as long as the substitution is chemically acceptable. However, provided that the number of substituents is two or more, they may be the same or different.

As "an optionally substituted amino group", an amino group optionally substituted by a lower alkyl group (described below), a lower alkanoyl group (for example, an alkanoyl group having 1 to 6 carbon atoms, such as formyl, acetyl, and propionyl) and the like, and the like can be mentioned.

As "an optionally substituted carboxyl group", a carboxyl group optionally substituted by a lower alkyl group (described below), a lower alkanoyl group (for example, an alkanoyl group having 1 to 6 carbon atoms, such as formyl, acetyl, and propionyl) and the like, and the like can be mentioned.

As "an optionally substituted amide group", an unsubstituted amide group, a substituted amide [N-substituted amide group or N,N'-di-substituted amide group; specifically, an amide group substituted by a lower alkyl group (described below), and the like] can be mentioned.

As "a halogen atom", fluorine, chlorine, bromine, and iodine can be mentioned.

As the ring that R' and R" may bind together to form in cooperation with the adjoining nitrogen atom, a saturated or unsaturated heterocyclic group (described below) comprising a nitrogen atom can be mentioned, and the ring is optionally substituted by a halogen atom (described above), a carboxyl group, a substituted amide group (described above), an optionally substituted lower alkyl group (described below) and the like.

As examples of "a saturated or unsaturated heterocyclic group", a 5- to 6-membered monocyclic group comprising one to two nitrogen atoms, a 5- to 6-membered monocyclic group comprising one to two nitrogen atoms and one oxygen atom or one sulfur atom, a 5-membered monocyclic group comprising one oxygen atom or one sulfur atom, a bicyclic group comprising one to four nitrogen atoms and resulting from the condensation of a 6-membered ring and a 5- or 6-membered ring, and the like can be mentioned; specifically, for example, pyridyl, thienyl, oxaziazolyl, imidazolyl, thiazolyl, isothiazolyl, oxazolyl, isoxazolyl, furyl, pyrrolyl, quinolyl, quinazolinyl, purinyl, pyrazolyl, thiophenyl and the like can be mentioned. The heterocyclic group is optionally substituted by a substituent such as a saturated or unsaturated cyclic hydrocarbon group (described below), a saturated or unsaturated heterocyclic group (described above), a halogen atom (described above), a cyano group, a nitro group, an oxo group, an optionally substituted carboxyl group (described above), a substituted amide group (described above), an optionally substituted lower alkyl group (described below), an optionally substituted amino group (described above), or an optionally substituted alkoxy group (described above); these substituents substitute on the heterocyclic group, as long as the substitution is chemically acceptable. Provided that the number of substituents is two or more, they may be the same or different.

"A lower alkyl group" denotes, for example, a linear, branched or cyclic alkyl group having 1 to 6 carbon atoms; specifically, methyl, ethyl, n-propyl, isopropyl, butyl, isobutyl, sec-butyl, tert-butyl, pentyl, hexyl, cyclopropyl, cyclobutyl and the like can be mentioned. As the "substituent" in "an optionally substituted lower alkyl group", a carboxyl group, a substituted amide group (having the same definition as described above), a cyano group, a hydroxyl group, a halogen atom (described above) and the like can be mentioned.

As examples of "an aroxy group", a 6-membered monocyclic group (for example, phenyl group) across an oxygen atom can be mentioned; specifically, for example, phenoxy and the like can be mentioned. The monocyclic group is optionally substituted by a substituent such as a saturated or unsaturated cyclic hydrocarbon group (described below), a saturated or unsaturated heterocyclic group (described above), a halogen atom (described above), a cyano group, a nitro group, an optionally substituted carboxyl group (described above), a substituted amide group (described above), an optionally substituted lower alkyl group (described below), an optionally substituted amino group (described above), or an optionally substituted alkoxy group (described above); these substituents substitute on the cyclic group, as long as the substitution is chemically acceptable. Provided that the number of substituents is two or more, they may be the same or different. Furthermore, the monocyclic group may have condensed with a saturated or unsaturated cyclic hydrocarbon group (described below) or with a saturated or unsaturated heterocyclic group (described above), to form a condensed ring. As the condensed ring, indene, naphthalene, fluorene, phenanthrene, anthracene, indole, isoindole, benzofuran, benzothiophene, indolizine, chromene, quinoline, isoquinoline, indazole, quinazoline, cinnoline, quinoxaline, phthalazine and the like can be mentioned.

"A saturated or unsaturated cyclic hydrocarbon group" denotes a saturated or unsaturated cyclic hydrocarbon group having 3 to 18 carbon atoms; specifically, for example, an alicyclic hydrocarbon group, an aromatic hydrocarbon group and the like can be mentioned.

As examples of the "alicyclic hydrocarbon group", a monocyclic or condensed polycyclic group comprising 3 to 10 carbon atoms, specifically, a cycloalkyl group, a cycloalkenyl group and a bicyclic or tricyclic condensed ring thereof with an aryl group having 6 to 14 carbon atoms (for example, benzene and the like) or the like, and the like can be mentioned. As examples of the "cycloalkyl group", a cycloalkyl group having 3 to 6 carbon atoms, such as cyclopropyl, cyclobutyl, cyclopentyl, and cyclohexyl can be mentioned; as examples of the "cycloalkenyl group", a cycloalkenyl group having 3 to 6 carbon atoms, such as cyclopropenyl, cyclobutenyl, cyclopentenyl, and cyclohexenyl, and the like can be mentioned.

As examples of the "aromatic hydrocarbon group", a monocyclic aromatic hydrocarbon group comprising 6 to 18 carbon atoms, a condensed polycyclic aromatic hydrocarbon group and the like can be mentioned; specifically, an aryl group having 6 to 14 carbon atoms, such as phenyl, 1-naphthyl, 2-naphthyl, 2-indenyl, and 2-anthryl, can be mentioned.

The cyclic hydrocarbon group is optionally substituted by a substituent such as a saturated or unsaturated cyclic hydrocarbon group (described above), a saturated or unsaturated heterocyclic group (described above), a halogen atom (described above), a cyano group, a nitro group, an oxo group, an optionally substituted carboxyl group (described above), a substituted amide group (described above), an optionally substituted lower alkyl group (described above), an optionally substituted amino group (described above), and an optionally substituted alkoxy group (described above); these substituents substitute on the cyclic hydrocarbon group, as long as the substitution is chemically acceptable. However, provided that the number of substituents is two or more, they may be the same or different.

The compound of the present invention, represented by the general formula (I) or the general formula (II), can be produced by applying various commonly known synthetic methods by means of the characteristics based on the basic skeleton thereof or the kind of substituent. For example, alkylation, acylation, amination, imination, halogenization, reduction, oxidation, condensation and the like can be mentioned, and reactions or methods in common use in the art can be utilized.

A compound capable of binding to MFP-2 or a functional fragment thereof, such as the compound of the present invention, represented by the general formula (I) or the general formula (II), exhibits excellent anti-inflammatory action and anti-allergic action in mammals such as monkeys, horses, cattle, sheep, dogs, cats, rabbits, mice, rats, and guinea pigs, including humans, and is therefore useful for the treatment of a disease selected from the group consisting of an inflammatory disease and an allergic disease.

The compound used in the present invention, which binds specifically to MFP-2 and exhibits anti-inflammatory action and/or anti-allergic action (hereinafter also referred to as the compound of the present invention), may have formed a pharmaceutically acceptable salt; as the salt, acid addition salts, for example, inorganic acid salts (for example, hydrochlorides, sulfates, hydrobromates, phosphates and the like), organic acid salts (for example, acetates, trifluoroacetates, succinates, malates, fumarates, propionates, citrates, tartrates, lactates, oxalates, methanesulfonates, p-toluenesulfonates and the like) and the like can be mentioned.

Note that the compound of the present invention or a salt thereof may be a solvate such as a hydrate.

When the compound of the present invention is used as a therapeutic drug for a disease selected from the group consisting of an inflammatory disease and an allergic disease, it is prepared as an ordinary pharmaceutical preparation and administered orally or parenterally.

For oral administration, the compound of the present invention can be administered in a dosage form in common use in the art. For parenteral administration, the compound of the present invention can be administered in a dosage form such as a topical preparation (transdermal preparation and the like), a rectal preparation, an injection, or a nasal preparation.

As examples of the oral preparation or rectal preparation, capsules, tablets, pills, powders, drops, cachets, suppositories, liquids and the like can be mentioned. As examples of the injection, a sterile solution or suspension and the like can be mentioned. As examples of the topical preparation, creams, ointments, lotions, transdermal preparations (ordinary patches, matrices) and the like can be mentioned.

The above-described dosage forms can be formulated along with a pharmaceutically acceptable excipient and additive by a technique commonly performed in the art. As the pharmaceutically acceptable excipient and additive, carriers, binders, flavoring agents, buffering agents, thickeners, colorants, stabilizers, emulsifiers, dispersing agents, suspending agents, antiseptics and the like can be mentioned.

As examples of pharmaceutically acceptable carriers, magnesium carbonate, magnesium stearate, talc, sugar, lactose, pectin, dextrin, starch, gelatin, gum tragacanth, methylcellulose, sodium carboxymethylcellulose, low-melting-point waxes, cacao butter and the like can be mentioned.

Furthermore, the tablets can be prepared as tablets with ordinary coatings, for example, sugar-coated tablets, enteric coated tablets, film-coated tablets, and double-layered tablets or multilayered tablets if necessary. The powders are formulated into preparations along with a pharmaceutically acceptable base for powders. As the base, talc, lactose, starch and the like can be mentioned. The drops can be formulated into preparations along with an aqueous or nonaqueous base and one kind or more of pharmaceutically acceptable diffusing agents, suspending agents, solubilizers and the like. The capsules can be produced by filling therein an active ingredient compound, along with a pharmaceutically acceptable carrier. The compound can be mixed with a pharmaceutically acceptable excipient and filled in the capsules, or filled without an excipient. The caches can also be produced in the same manner. When the present invention is prepared as a suppository, it is formulated into preparations by a commonly used technique along with a base such as a vegetable oil (castor oil, olive oil, peanut oil and the like), a mineral oil (petrolatum, white petrolatum and the like), a wax, or a partially synthesized or totally synthesized glycerine fatty acid ester.

As the liquid for injection, solutions, suspensions, emulsions and the like can be mentioned. For example, aqueous solutions, water-propylene glycol solutions and the like can be mentioned. The liquid can also be produced in the form of a solution of polyethylene glycol and/or propylene glycol that may contain water.

A liquid suitable for oral administration can be produced by adding an active ingredient compound to water and, if required, adding a colorant, flavoring agent, stabilizer, sweetener, solubilizer, thickener and the like. A liquid suitable for oral administration can also be produced by adding the compound, along with a dispersing agent, to water to increase the viscosity. As examples of the thickener, pharmaceutically acceptable natural or synthetic rubbers, resins, methylcellulose, sodium carboxymethylcellulose, known suspending agents and the like can be mentioned.

As the topical preparation, the above-described liquids, as well as creams, aerosols, sprays, dusting powders, lotions, ointments and the like can be mentioned. The above-described topical preparation can be produced by mixing an active ingredient compound and pharmaceutically acceptable diluent and pharmaceutically acceptable carrier. Ointments and creams are prepared by, for example, adding a thickener and/or a gelling agent to an aqueous or oily base. As examples of the base, water, liquid paraffin, vegetable oils and the like can be mentioned. As examples of the thickener, soft paraffin, aluminum stearate, cetostearyl alcohol, propylene glycol, polyethylene glycol, lanolin, hydrogenated lanolin, beeswax and the like can be mentioned. To the topical preparation, an antiseptic such as methyl hydroxybenzoate, propyl hydroxybenzoate, chlorocresol, or benzalkonium chloride, and a bacterial growth inhibitor can be added. A lotion can be prepared by adding one or more kinds of pharmaceutically acceptable stabilizers, suspending agents, emulsifiers, diffusing agents, thickeners, colorants, flavoring agents and the like to an aqueous or oily base.

Dosage and frequency of administration vary depending on the kind of compound used, symptoms, age and body weight of patients, dosage form and the like, and are set as appropriate according to them.

The present invention also enables screening for a compound useful for the treatment of a disease such as an inflammatory disease or an allergic disease, with specific bindability to MFP-2 or a functional fragment thereof (the definitions for the individual terms are as described above) as an index. Here, MFP-2 or a functional fragment thereof can be used as a purified or unpurified protein (polypeptide) or a (functional) fragment thereof, and can be used in the state of being expressed in cells. MFP-2 or a (functional) fragment thereof can be acquired by using as appropriate a known technique such as (1) a method comprising isolation and purification from a cell culture or tissue, as a starting material, producing MFP-2 or a (functional) fragment thereof, (2) a method comprising chemical synthesis, or (3) a method comprising purification from cells manipulated by gene recombination technology and the like to express MFP-2 or a (functional) fragment thereof.

Isolation and purification of the MFP-2 or a (functional) fragment thereof of the present invention can, for example, be performed as described below. That is, the MFP-2 or a (functional) fragment thereof is extracted and purified by a known method from a tissue expressing MFP-2 or a (functional) fragment thereof, or a culture obtained by culturing cells expressing MFP-2 or a (functional) fragment thereof in an appropriate liquid medium. For the extraction and purification, known methods are used as appropriate depending on the fraction wherein the desired product is present.

Specifically, the extraction and purification are performed as described below. First, a tissue or culture is directly subjected to a conventional method such as filtration or centrifugation, and the tissue or cells or the supernatant is recovered. If the desired protein has been accumulated in the cells, the recovered cells are suspended in an appropriate buffer solution, and a surfactant is added at an appropriate concentration to solubilize the membrane. As the surfactant, sodium dodecyl sulfate (SDS), cetyltrimethylammonium bromide (CTAB) and the like can be mentioned. Since these exhibit potent protein denaturing action, it is preferable to use a gently acting nonionic surfactant, for example, Triton X-100 and the like, to ensure that the protein is folded to show biological activity. Next, the crude extract obtained is treated in the presence of a surfactant if required, using commonly used methods in combination as appropriate, to isolate and purify the protein or a functional fragment thereof. As such methods, methods based on differences in solubility, such as salting-out and solvent precipitation; methods based on differences in molecular weight, such as dialysis, ultrafiltration, gel filtration, and SDS-PAGE; methods based on electric charge, such as ion exchange chromatography; methods based on specific affinity, such as affinity chromatography; methods based on differences in hydrophobicity, such as reverse phase high performance liquid chromatography; methods based on differences in isoelectric point, such as isoelectric focusing; and the like can be mentioned. More specifically, the protein or a functional fragment thereof can be separated and purified by commonly used methods, for example, concentration under reduced pressure, lyophilization, extraction with conventionally used solvents, pH adjustment, treatment with conventionally used adsorbents such as anion exchange resin or cation exchange resin, and nonionic adsorption resin, crystallization, recrystallization and the like.

Production of the MFP-2 or a (functional) fragment thereof of the present invention by chemical synthesis can be performed by, for example, synthesis or semi-synthesis based on the amino acid sequence information shown by SEQ ID NO:2 using a peptide synthesizer.

Also, when the MFP-2 or a (functional) fragment thereof is acquired from cells manipulated to express the same by gene recombination technology and the like, the specific procedures are performed as described below.

First, an expression vector that functionally carries the gene encoding MFP-2 or a functional fragment thereof is prepared.

The gene that encodes MFP-2 or a functional fragment thereof may be obtained by any method. For example, a complementary DNA (cDNA) prepared from an mRNA, a genomic DNA prepared from a genomic library, a chemically synthesized DNA, a DNA obtained by amplification by the PCR method with an RNA or DNA as a template, and a DNA constructed by appropriately combining these methods, and the like are included. For example, a DNA comprising all or a portion of a DNA substantially comprising the base sequence shown by SEQ ID NO:1 (Accession No. X94978), particularly the base sequence shown by the base numbers 28 to 2232, a DNA comprising all or a portion of a DNA substantially comprising the base sequence shown by SEQ ID NO:3 (Accession No. X87176), particularly the base sequence shown by the base numbers 49 to 2259, a DNA comprising all or a portion of a DNA substantially comprising the base sequence shown by SEQ ID NO:5 (Accession No. X89998), particularly the base sequence shown by the base numbers 13 to 2220, and the like can be mentioned. Also, a technology for substituting or deleting an optionally chosen base in the above-described base sequence (for example, in vitro mutagenesis, site-directed mutagenesis and the like) can also be utilized.

As used herein, "a DNA substantially comprising" means, in addition to the above-described DNAs comprising a particular base sequence, a DNA comprising a base sequence capable of hybridizing to the above-described DNAs comprising a particular base sequence under stringent conditions (in the present invention, these conditions refer to conditions under which a DNA having a homology of about 60% or more, preferably about 80% or more, and more preferably about 90% or more, in terms of base sequence can hybridize; stringency can be controlled by changing the temperature, salt concentration and the like as appropriate during the hybridization reaction and washing). Stringent conditions can be calculated on the basis of the desired homology, the length of oligonucleotide and the like by applying them to appropriate calculation formulas utilized in the art. For example, hybridization at 42°C and washing treatment at 42°C with a buffer solution containing 1×SSC and 0.1% SDS, hybridization at 65°C and washing treatment at 65°C with a buffer solution containing 0.1×SSC and 0.1% SDS, and the like can be mentioned.

An expression vector that functionally comprises a gene encoding MFP-2 or a functional fragment thereof can be obtained by inserting the DNA obtained into a plasmid vector, phage vector or the like capable of retaining replication or autonomous replication in various hosts of prokaryotic cells and/or eukaryotic cells by means of an appropriate restriction endonuclease site.

As used herein, "functionally" means that the gene (DNA) is arranged to allow transcription in a host cell matching with the vector, and to allow the production of the protein encoded thereby. Preferably, the expression vector is a vector having an expression cassette wherein a promoter region, an initiation codon, a gene encoding MFP-2 or a functional fragment thereof, a stop codon and a terminator region are continuously arranged. For transformant selection, it is preferable that a selection marker gene be further contained.

For example, when a mammalian cell is transformed, a plasmid comprising a promoter and a polyadenylation signal both of an animal virus, for example, SV40, RSV, MMLV and the like, , joined to each other via a restriction endonuclease site, preferably a multicloning site, wherein a selection marker gene derived from a plasmid such as pSV2-neo or pSV2-dhfr (neomycin resistance gene, dihydrofolate reductase and the like) has been inserted, can be used.

The host cell is not subject to limitation, as long as it matches with the expression vector used, and is transformable; various cells in common use in the technical field of the present invention, such as natural cells or an artificially established line of recombinant cells and the like, can be utilized. Specifically, bacteria such as *Escherichia coli* and *Bacillus subtilis,* fungi such as yeast, animal cells or insect cells and the like can be mentioned as examples. Preferably, mammalian cells, particularly rat-derived cells, hamster-derived cells (CHO, BHK and the like), mouse-derived cells (COP, L, C127, Sp2/0, NS-1, NIH T3 and the like), monkey-derived cells (COS1, COS3, COS7, CV1, Velo and the like) and human-derived cells (Hela, diploid fibroblast-derived cells, myeloma cells, Namalwa, Jurkat cells and the like) can be mentioned.

Introduction of an expression vector to a host cell can be performed using a conventionally known method. For example, when the expression vector is introduced to a mammalian cell, the calcium phosphate co-precipitation method, the protoplast fusion method, the microinjection method, the electroporation method, the lysosome method and the like can be mentioned.

MFP-2 or a functional fragment thereof can also be produced by culturing a transformant comprising an expression vector prepared as described above. The medium preferably contains a carbon source and inorganic or organic nitrogen source required for the growth of the host cell (transformant). As examples of the carbon source, glucose, dextrin, soluble starch, sucrose and the like can be mentioned; as examples of the nitrogen source, ammonium salts, nitrates, amino acids, corn steep liquor, peptone, casein, meat extract, soybean cake, potato extract and the like can be mentioned. If desired, other nutrients [for example, inorganic salts (calcium chloride, sodium dihydrogen phosphate, magnesium chloride and the like), vitamins, antibiotics (tetracycline, neomycin, kanamycin, ampicillin and the like)] may be contained.

The cultivation is performed by a method known in the art. The cultivation conditions are conditions enabling the expression of the protein; for example, temperature, medium pH and cultivation time are chosen as appropriate so that the protein is produced in a large amount.

For example, when the host is an animal cell, as examples of the medium, a minimum essential medium (MEM) containing about 5 to 20% fetal calf serum (FCS), Dulbecco's modified Eagle medium (DMEM), RPMI-1640 medium, 199 medium and the like can be used. The pH of the medium is preferably about 6 to 8, the cultivation is normally performed at 30 to 40°C for about 15 to 72 hours, and the culture may be aerated or agitated as necessary.

The MFP-2 or a functional fragment thereof of the present invention can be collected from the culture obtained from the above-described cultivation in the same manner as the aforementioned extraction, isolation, and purification from cells or tissue expressing MFP-2 or a functional fragment thereof.

Contact treatment of the MFP-2 or functional fragment thereof thus obtained and a test compound can be performed in accordance with a binding experiment commonly performed in the art. Specifically, in cases where MFP-2 or a functional fragment thereof or a test compound is immobilized to a solid phase carrier, a solution comprising the test compound is brought into contact with the solid phase carrier when the MFP-2 or a functional fragment thereof is immobilized, and a solution comprising the MFP-2 or a functional fragment thereof (a purified protein solution or a crudely purified protein solution such as cell extract or tissue extract) is brought into contact with the solid phase carrier when the test compound is immobilized to the solid phase carrier. The column method, the batch method and the like can be utilized.

The step for determining whether or not the test compound binds specifically to MFP-2 or a functional fragment thereof can be changed as appropriate depending on how the step for bringing the test compound into contact with MFP-2 or a functional fragment thereof has been performed; for example, when using a column packed with a solid phase carrier (for example, bead resin) immobilized with the test compound, MFP-2 molecules bind onto the solid phase carrier with the subsequent addition of a solution (sample) comprising MFP-2 or a functional fragment thereof, provided that there is specific affinity between the two (do not bind in the absence of specific affinity). It is also possible to dissociate the bound MFP-2 or a functional fragment thereof from the solid phase by a treatment such as altering the polarity of the buffer solution or further adding the test compound in excess, and then identify, or to extract with a surfactant and the like while remaining in a state bound to the test compound on the solid phase, and then identify. As the method of identification, specifically, known techniques such as electrophoresis, immunoblotting and immunoprecipitation, which employ immunological reactions, chromatography, mass spectrometry, amino acid sequencing, and NMR, or combinations of these methods can be used. By determining whether or not MFP-2 or a functional fragment thereof is captured onto the solid phase or contained in the column through fraction, or the extent thereof and the like, a judgment is made as to whether or not the test compound is capable of binding specifically to MFP-2, and a binding compound is selected.

Also, this step may be automated. For example, it is also possible to directly read data on various molecules obtained by two-dimensional electrophoresis, and identify the molecules on the basis of existing databases.

Furthermore, when MFP-2 or a functional fragment thereof is used in the state of being expressed in cells, it is also possible to determine the presence or absence of binding of MFP-2 or a functional fragment thereof and the test compound, and the degree of binding, by making use of various labeling techniques such as RI labeling and fluorescence labeling. "Contact of MFP-2 or a functional fragment thereof and a test compound" in the screening method of the present invention also includes this mode. The contact conditions of cells and a test compound are set as appropriate depending on factors such as the cells used and the expression status of MFP-2 or a functional fragment thereof in the cells. Also, whether or not MFP-2 or a functional fragment thereof is expressed in the cells is preferably confirmed in advance using an antibody and the like.

### Examples

The present invention is hereinafter described in more detail by means of the following Examples, which, however, are not to be construed as limiting the scope of the invention. Also, the compounds, reagents and the like used are commercially available or can be prepared on the basis of known reports and the like, unless otherwise specified.

### Example 1: Synthesis of Intal-immobilized resin

(1) Synthesis of 1,3-bis(2-carboxychromon-5-yloxy)-2-hydroxypropane 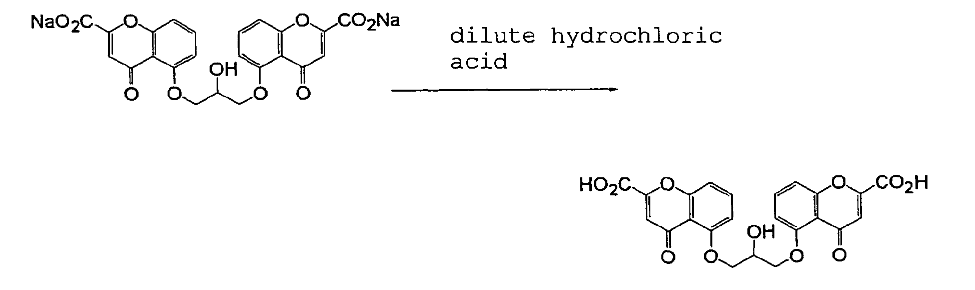
   The disodium salt of 1,3-bis(2-carboxychromon-5-yloxy)-2-hydroxypropane (Intal; 1 g) was dissolved in 50 ml of water. Dilute hydrochloric acid was added to obtain a pH of 3. The precipitated white crystal was collected by filtration and washed with water, ethanol, and ether. The crystal was dried under reduced pressure to yield a white crystal of 1,3-bis(2-carboxychromon-5-yloxy)-2-hydroxypropane (600 mg, yield 66%). ¹H-NMR (DMSO-d₆) δ: 4.30 (4H, d), 4.36 (1H, t), 5.32 (1H, bs), 6.86 (2H, s), 7.11 (2H, d), 7.17 (2H, d), 7.71 (2H, t).
(2) Synthesis of 1,3-bis(2-carboxychromon-5-yloxy)-2-hydroxypropane-immobilized resin 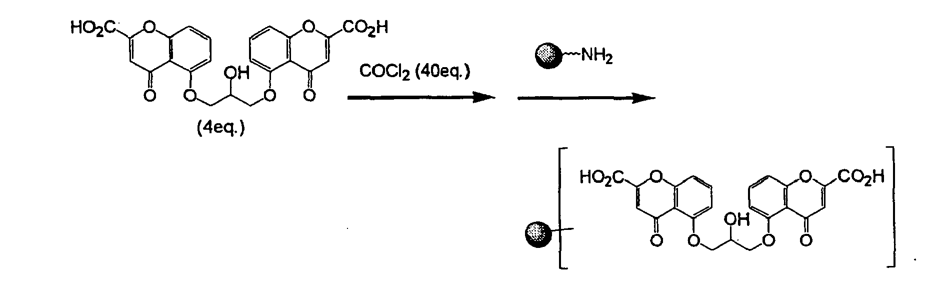
   1) Immobilization of 1,3-bis(2-carboxychromon-5-yloxy)-2-hydroxypropane
      To an acetonitrile suspension (0.5 ml) of 1,3-bis(2-carboxychromon-5-yloxy)-2-hydroxypropane (18.7 mg, 0.04 mmol), a toluene solution of phosgene (1.45 mol/l, 275 µl) was added. After the reaction at room temperature for 30 minutes, the mixture was heated at 70°C for 1 minute. The reaction solution of 1,3-bis(2-carboxychromon-5-yloxy)-2-hydroxypropane and phosgene was added to an acetonitrile suspension (0.2 ml) of TOYO-Pearl resin (TSKgel AF-amino, 100 µl, free amino group (available amino group) content 0.01 mol), and diisopropylethylamine (2.1 µl, 0.012 mmol), and the reaction was allowed to proceed at room temperature overnight. Next, the TOYO-Pearl resin was washed. The resin was washed with acetonitrile, dimethylformamide, water, and dimethylformamide five times each in this order. Residual amino groups were determined by the ninhydrin reaction, and the reaction ratio was calculated to be 43%. The resin was washed with a saturated aqueous solution of sodium hydrogen carbonate, water, and dimethylformamide five times each.
   2) Stearic acid and acetyl capping
      A dimethylformamide (DMF) suspension of TOYO-Pearl resin, stearic acid (6.5 mg, 0.023 mmol), 1-(3-dimethylaminopropyl)-3-ethylcarbodiimide (4.7 µl, 0.027 mmol), and 1-hydroxybenzotriazole (HoBt; 3.6 mg, 0.027 mmol) was reacted at room temperature overnight. The TOYO-Pearl resin was washed with DMF five times. Residual amino groups were determined by the ninhydrin reaction, and the reaction ratio was calculated to be 99%. To the TOYO-Pearl resin, acetic anhydride (200 µl) and DMF (800 µl) were added. After the reaction was allowed to proceed at room temperature for 1 hour, the resin was washed with DMF five times. It was confirmed that residual amino groups were no longer macroscopically observable by the ninhydrin reaction. At this time, the reaction ratio was calculated to be 99%. Finally, the TOYO-Pearl resin was washed with a 20% aqueous solution of ethanol five times.

### Example 2: Synthesis of tiaramide-immobilized resin

(1) Synthesis of TOYO-Pearl resin conjugated with tiaramide 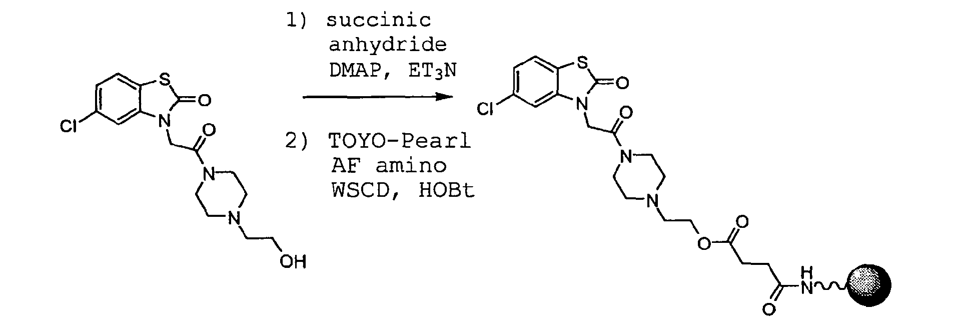
   Tiaramide (36 mg, 0.1 mmol) was dissolved in 1 ml of dichloromethane, and the solution was stirred at room temperature. Succinic anhydride (12 mg, 0.12 mmol), a catalytic amount of 4(N,N-dimethylamino)pyridine (DMAP; 4 mg), and triethylamine (Et₃N; 12.1 mg, 0.12 mmol) were added, and the solution was stirred at room temperature for 3 hours. The carboxylic acid obtained was added to 5 ml of a DMF suspension of TOYO-Pearl (AF-amino) (600 µl, 60 µmol), and condensed with water-soluble carbodiimide (WSCD; 21 µl, 120 µmol) and HOBt (17.8 mg, 132 µmol). After stirring at room temperature for one day, the resin was washed with DMF five times, and the ninhydrin test was performed; as a result, the desired compound was obtained with a yield of about 97%. 5 ml of a 20% DMF solution of acetic anhydride was added, and the solution was stirred at room temperature for 30 minutes to cap the remaining amino groups with acetyl groups. The resin was washed with 20% ethanol to yield desired TOYO-Pearl resin conjugated with tiaramide.

### Example 3: Binding experiments

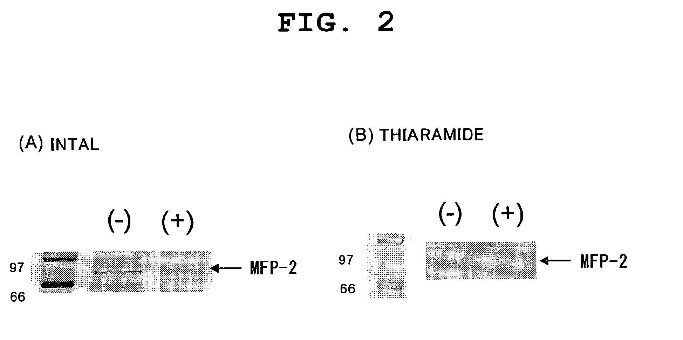

(1) Preparation of rat brain lysate
   The rat brain (2.4 g) was mixed in a mixed solution A (25 mM Tris-HCl pH 8.0, 0.5% Tween 20, 300 µM DCC (24 ml; N,N-diethyldithiocarbamate sodium)) and prepared as a homogenate, which was then centrifuged at 9,000 rpm for 10 minutes. The centrifugal supernatant was collected and further centrifuged at 50,000 rpm for 30 minutes. The supernatant thus obtained was used as the lysate. Note that all experiments were performed at 4°C or on ice.
(2) Binding experiments
   Binding experiments were performed using the immobilizing resins with each test compound immobilized thereon, prepared in Examples 1 to 2, and the rat brain lysate prepared in Example 3(1), per the procedures shown below.
   Each resin (10 µl) and lysate (1 ml) were gently shaken at 4°C for about 1 hour. Thereafter, centrifugal operation was performed, and each supernatant was collected carefully. Then, each supernatant was again mixed with a fresh compound-bound resin (10 µl). At this time, the separated compound-bound resin was gently stored at 4°C as the resin of the first binding experiment. After the mixture was gently stirred for about 1 hour, centrifugal operation was performed, and the supernatant was removed. Subsequently, the compound-bound resin obtained in the second binding experiment and the resin obtained in the first binding experiment were carefully washed with mixed solution A about five times each to remove substances other than the protein bound onto the resin to the maximum possible extent. To each compound-bound resin thus obtained, 25 µl of a loading buffer for SDS (nakalai Cat.NO = 30566-22, sample buffer solution for electrophoresis with 2-ME (2-mercaptoethanol) (2x) for SDS PAGE) was added; this was followed by stirring at 25°C for 10 minutes. The sample solution thus obtained was separated using a commercially available SDS gel (BioRad readyGel J, 10% SDS, cat. NO=161-J341), and the SDS gel was analyzed (Figure 2). An electrophoregram of the sample solution comprising the protein bound onto the binding resin obtained in the first binding experiment (in Figure 2, denoted as (-) for the sake of convenience), and an electrophoregram of the sample solution comprising the protein bound onto the binding resin obtained in the second binding experiment (in Figure 2, denoted as (+) for the sake of convenience) were compared.
   As a result, MFP-2 commonly bound to the Intal- and tiaramide-immobilized resins, and the binding was remarkably confirmed in the first binding experiment with each compound-bound resin but minimally observed in the second binding experiment; therefore, the binding was shown to be a specific binding.
   Also, the subject band on the SDS gel was cut out and subjected to in-gel digestion with trypsin according to a commonly used protocol (Hisaaki Taniguchi, Miki Kikuchi, Saibo Kogaku, 21(5), p524 (2002)), after which the digested peptide fragment was examined by fingerprinting analysis using MALDI-TOF mass spectrometry to confirm that the subject protein was MFP-2. The protein search software used was Mascot.

### Example 4: Measurement of Kd value between MFP-2 and Intal using Biacore

### <Synthesis of Intal-immobilized chip>

The SIA kit Au from Biacore Company (#BR-1004-05) was immersed in Piranha solution (25% H₂O₂, 75% H₂SO₄) in a glass dish, and washed with shaking for 3 hours. Next, this chip was washed with Milli-Q Water and ethanol, and allowed to stand overnight as immersed in an ethanol solution (1.5 mM) of 11-amino-1-undecanethiol hydrochloride (Dojindo, #A423). After this was washed with N-methylpyrrolidone (NMP), it was placed in an NMP solution containing N-Fmoc-amido-dPEG₄™-acid (50 mM), PyBop (50 mM) and diisopropylethylamine (Pr₂Net; 100 mM) and shaken for 16 hours to cause the reaction. After the mixture was washed with NMP, the reaction was allowed to proceed in an acetonitrile (MeCN) solution containing acetic acid (10 mM), HOBt (10 mM) and WSCI (water-soluble carbodiimide; 10 mM) for 5.5 hours. Furthermore, the mixture was reacted with a 20% piperidine/MeCN mixed solution for 30 minutes to achieve Fmoc elimination. Furthermore, after washing with MeCN, 70 µl of an Intal immobilization reaction solution (1.05 µM Intal, 10.5 µM HOBt, 10.5 µM WSCI) was added onto the gold membrane, and the membrane was allowed to stand overnight to immobilize the Intal.

To achieve acetyl capping after the reaction, the reaction was performed again in an MeCN solution containing acetic acid (10 mM), HOBt (10 mM), and WSCI (10 mM) for 5.5 hours to achieve capping and finish the gold membrane.

Note that control data were obtained by using a gold thin membrane chip prepared in the same manner as described above, but skipping the reaction process with the above-described Intal immobilization solution.

### <MFP-2 expression>

An entry vector was prepared using the GATEWAY system from Invitrogen Company, and recombinant MFP-2 was expressed as a protein with N-terminal His-tag using an E. coli expression system (pDEST17). Ni-NTA purification (QIAGEN Company) and gel filtration purification (Amersham Bioscience, Superdex 200 10/300) were performed to obtain a purity of not less than 70%. The purified MFP-2 was used for measurements after buffer exchange with a running buffer (25 mM Tris-HCl, 1% CHAPS, 150 mM NaCl).

### <Measurement of Kd value>

The Kd value was measured using Biacore 3000. The conditions were as follows: flow rate 40 µl/min; injection volume 20 µl; dissociation time 60 sec; MFP-2 concentrations 25, 12.5, and 6.25 nM.

After data were measured, differences from the control were determined using analytical software (BIACORE Company, BIA evaluation ver 4.1) and analyzed by global fitting to calculate the Kd value. As a result, the value was determined to be 0.64 nM.

From the fact above, it was demonstrated that the interaction between Intal and MFP-2 is a specific binding.

### Industrial Applicability

Whereas ordinary NSAIDs exhibit cyclooxygenase inhibition as their essential action, a compound obtained by the screening method of the invention of this application, or the compound of the invention of this application and a pharmaceutical composition comprising the compound exhibit anti-inflammatory action and anti-allergic action by different action mechanisms. Therefore, it is possible to provide a drug that supplements the therapeutic effects of cyclooxygenase inhibitors, which produce severe adverse reactions such as gastrointestinal disorder, or that replace them.

This application is based on a patent application No. 2004-156614 filed in Japan, the contents of which are incorporated in full herein by this reference.

## Claims

1. A pharmaceutical composition comprising as an active ingredient a compound that specifically binds to a protein having the amino acid sequence of SEQ ID NO:2.

2. A pharmaceutical composition comprising, as an active ingredient, a compound that specifically binds to a protein, which is **characterized by** having an amino acid sequence resulting from the deletion, substitution or addition of one or more amino acids in the amino acid sequence of SEQ ID NO:2 and binding to a compound of the formula (1) and/or a compound of the formula (2).

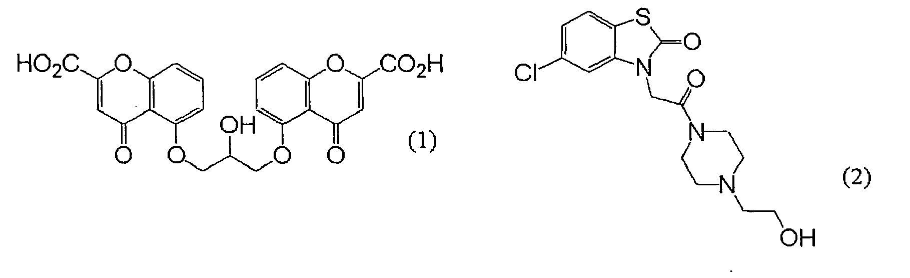

3. The pharmaceutical composition of claim 1 or 2, which is for the treatment of a disease selected from the group consisting of an inflammatory disease and an allergic disease.

4. A pharmaceutical composition comprising as an active ingredient a compound that binds specifically to MFP-2.

5. A pharmaceutical composition comprising as an active ingredient a compound that regulates the expression of MFP-2.

6. A pharmaceutical composition comprising as an active ingredient a compound that regulates the activity of MFP-2.

7. The pharmaceutical composition of any one of claims 4 to 6, which is for the treatment of a disease selected from the group consisting of an inflammatory disease and an allergic disease.

8. A method for screening for a compound useful for the treatment of a disease selected from the group consisting of an inflammatory disease and an allergic disease, which comprises the following steps of
(1) bringing MFP-2 or a functional fragment thereof into contact with a test compound,
(2) determining whether or not the test compound specifically binds to MFP-2 or a functional fragment thereof, and
(3) selecting a test compound that specifically binds to MFP-2 or a functional fragment thereof in the step (2) above.

9. A method for screening for a compound useful for the treatment of a disease selected from the group consisting of an inflammatory disease and an allergic disease, which comprises the following steps of
(1) bringing a protein having the amino acid sequence of SEQ ID NO:2 or a functional fragment thereof into contact with a test compound,
(2) determining whether or not the test compound specifically binds to the protein or a functional fragment thereof, and
(3) selecting a test compound that specifically binds to the protein or a functional fragment thereof in the step (2) above.

10. A method for screening for a compound useful for the treatment of a disease selected from the group consisting of an inflammatory disease and an allergic disease, which comprises the following steps of
(1) bringing a protein, which is **characterized by** having an amino acid sequence resulting from the deletion, substitution or addition of one or more amino acids in the amino acid sequence of SEQ ID NO:2 and binding to a compound of the formula (1) and/or a compound of the formula (2), or a functional fragment thereof, into contact with a test compound, 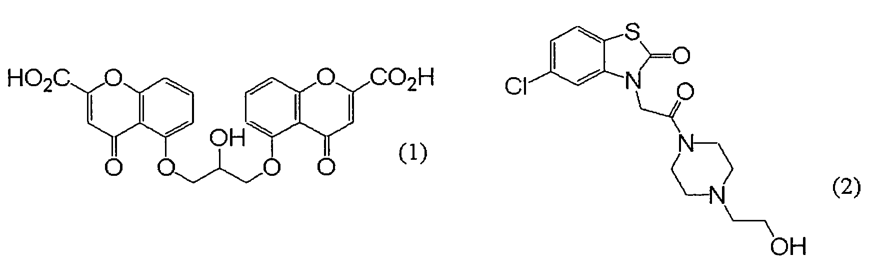
(2) determining whether or not the test compound specifically binds to the protein or a functional fragment thereof, and
(3) selecting a test compound that specifically binds to the protein or a functional fragment thereof in the step (2) above.

11. A compound useful for the treatment of a disease selected from the group consisting of an inflammatory disease and an allergic disease, obtained by the screening method of any one of claims 8 to 10.

12. A compound represented by the general formula (I) or the general formula (II) or a pharmaceutically acceptable salt thereof: 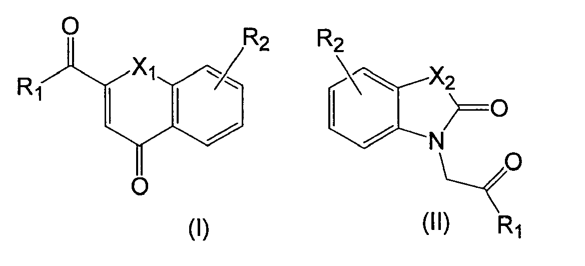 (wherein X₁ is O, NH, NR'", S, SO or SO₂;
X₂ is O, NH, NR'", S, -CH₂CH₂- or -CH=CH-;
R₁ is H, OH, OR', NH₂, NHR' or NR'R'';
R₂ is an optionally substituted saturated or unsaturated chain-structured hydrocarbon group, an optionally substituted alkoxy group, an optionally substituted amino group, an optionally substituted carboxyl group, an optionally substituted amide group or a halogen atom;
R', R" and R'" are the same or different and each is an optionally substituted saturated or unsaturated chain-structured hydrocarbon group;
provided that R₁ is NR'R", R' and R" may bind together to form a ring in cooperation with the adjoining nitrogen atom),
but the compounds represented by the formula (1) and the formula (2) are excluded.

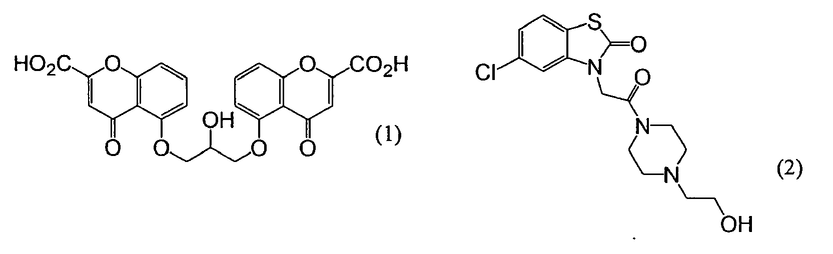

13. A pharmaceutical composition comprising as an active ingredient the compound of claim 12 or a pharmaceutically acceptable salt thereof.

14. The pharmaceutical composition of claim 13, which is for the treatment of a disease selected from the group consisting of an inflammatory disease and an allergic disease.
